# EUROPEAN PATENT APPLICATION

(11) **EP 4 538 289 A1**
(43) Date of publication of application: **16.04.2025**
(21) Application number: 23819136.5
(22) Date of filing: 07.06.2023
(51) Int. Cl.: C07K 16/28, A61K 39/395, A61P 35/00

(54) **CD73 ANTIBODY COCKTAIL THERAPY**

(30) Priority: 08.06.2022 CN 202210639331
(71) Applicant: Shanghai Huaota Biopharmaceutical Co., Ltd., Shanghai 201203 (CN); Huabo Biopharm (Shanghai) Co., Ltd., Shanghai 201203 (CN)
(72) Inventor: XU, Jingen, Shanghai 201203 (CN); JIA, Huifeng, Shanghai 201203 (CN); ZHAN, Yifan, Shanghai 201203 (CN); PAN, Xianfei, Shanghai 201203 (CN); YU, Haijia, Shanghai 201203 (CN); WANG, Shaojie, Shanghai 201203 (CN); XIA, Simin, Shanghai 201203 (CN); ZHU, Xiangyang, Shanghai 201203 (CN)
(74) Representative: Weickmann & Weickmann PartmbB
(86) International application number: PCT/CN2023/098753
(87) International publication number: WO 2023/236971

(57) **Abstract**

A pharmaceutical combination, comprising a first antigen-binding protein that specifically binds to CD73, and a second antigen-binding protein that specifically binds to CD73. The first antigen-binding protein and the second antigen-binding protein have different CD73 antigen binding epitopes. Also provided are a pharmaceutical composition comprising the pharmaceutical combination and a use thereof.

## Description

### TECHNICAL FIELD

The present application relates to the field of biomedicine, and particularly to a CD73 antibody cocktail therapy.

### BACKGROUND

CD73, also referred to as NT5E, is an extracellular 5-nucleotidase (NT5E) with a molecular weight of about 70 Kd, which is anchored to the cell surface by glycosylphosphatidylinositol (GPI), and is mainly present in the form of a dimer. Under normal physiological conditions, CD73 can be expressed in various tissues or organs, such as liver, large intestine, kidney, spleen, lung, ovary, and lymph nodes.

A large number of preclinical research results show that CD73 is abnormally and highly expressed on the surfaces of various tumor cells, including cerebral glioma, breast cancer, melanoma, non-small cell lung cancer, bladder cancer, ovarian cancer, colorectal cancer, etc., and clinical data show that the high expression of CD73 is closely related to the poor prognosis of tumor patients, so that CD73 can be used as a potential target for clinical treatment and prognosis of various tumors.

Therefore, there is an urgent need to develop diverse therapies targeting CD73, which provide more new ideas for preventing and/or treating diseases caused by abnormal expression of CD73.

### SUMMARY

The present application provides a pharmaceutical combination comprising a first antigen-binding protein that specifically binds to CD73, and a second antigen-binding protein that specifically binds to CD73, wherein the first antigen-binding protein and the second antigen-binding protein have different CD73 antigen-binding epitopes. The pharmaceutical combination of the present application has one or more of the following properties: (1) being capable of specifically binding to CD73 protein; (2) having relatively high affinity for the CD73 protein; and (3) being capable of inhibiting the growth and/or proliferation of tumor cells.

In certain embodiments, the first antigen-binding protein and the second antigen-binding protein bind to the CD73 protein in a non-competitive manner.

In certain embodiments, the first antigen-binding protein and the second antigen-binding protein are present in the form of a mixture.

In certain embodiments, the first antigen-binding protein comprises at least one CDR in an antibody heavy chain variable region VH, the VH comprising the amino acid sequence set forth in SEQ ID NO: 8.

In certain embodiments, the first antigen-binding protein comprises HCDR3, the HCDR3 comprising the amino acid sequence set forth in SEQ ID NO: 1.

In certain embodiments, the first antigen-binding protein comprises HCDR2, the HCDR2 comprising the amino acid sequence set forth in SEQ ID NO: 2.

In certain embodiments, the first antigen-binding protein comprises HCDR1, the HCDR1 comprising the amino acid sequence set forth in SEQ ID NO: 3.

In certain embodiments, the first antigen-binding protein comprises a VH, the VH comprising the amino acid sequence set forth in SEQ ID NO: 8.

In certain embodiments, the first antigen-binding protein comprises an antibody heavy chain constant region.

In certain embodiments, the antibody heavy chain constant region is derived from an IgG heavy chain constant region.

In certain embodiments, the antibody heavy chain constant region is derived from a human IgG heavy chain constant region.

In certain embodiments, the antibody heavy chain constant region comprises the amino acid sequence set forth in SEQ ID NO: 9.

In certain embodiments, the first antigen-binding protein comprises an antibody heavy chain, the antibody heavy chain comprising the amino acid sequence set forth in SEQ ID NO: 10.

In certain embodiments, the first antigen-binding protein comprises at least one CDR in an antibody light chain variable region VL, the VL comprising the amino acid sequence set forth in SEQ ID NO: 18.

In certain embodiments, the first antigen-binding protein comprises LCDR3, the LCDR3 comprising the amino acid sequence set forth in SEQ ID NO: 11.

In certain embodiments, the first antigen-binding protein comprises LCDR2, the LCDR2 comprising the amino acid sequence set forth in SEQ ID NO: 12 (WAS).

In certain embodiments, the first antigen-binding protein comprises LCDR1, the LCDR1 comprising the amino acid sequence set forth in SEQ ID NO: 13.

In certain embodiments, the first antigen-binding protein comprises a VL, the VL comprising the amino acid sequence set forth in SEQ ID NO: 18.

In certain embodiments, the first antigen-binding protein comprises an antibody light chain constant region.

In certain embodiments, the antibody light chain constant region is derived from a human Igκ constant region.

In certain embodiments, the antibody light chain constant region comprises the amino acid sequence set forth in SEQ ID NO: 19.

In certain embodiments, the first antigen-binding protein comprises an antibody light chain, the antibody light chain comprising the amino acid sequence set forth in SEQ ID NO: 20.

In certain embodiments, the second antigen-binding protein comprises at least one CDR in an antibody heavy chain variable region VH, the VH comprising the amino acid sequence set forth in SEQ ID NO: 27.

In certain embodiments, the second antigen-binding protein comprises HCDR3, the HCDR3 comprising the amino acid sequence set forth in SEQ ID NO: 21.

In certain embodiments, the second antigen-binding protein comprises HCDR2, the HCDR2 comprising the amino acid sequence set forth in SEQ ID NO: 22.

In certain embodiments, the second antigen-binding protein comprises HCDR1, the HCDR1 comprising the amino acid sequence set forth in SEQ ID NO: 23.

In certain embodiments, the second antigen-binding protein comprises a VH, the VH comprising the amino acid sequence set forth in SEQ ID NO: 27.

In certain embodiments, the second antigen-binding protein comprises an antibody heavy chain constant region.

In certain embodiments, the antibody heavy chain constant region is derived from an IgG heavy chain constant region.

In certain embodiments, the antibody heavy chain constant region is derived from a human IgG heavy chain constant region.

In certain embodiments, the antibody heavy chain constant region of the second antigen-binding protein comprises the amino acid sequence set forth in SEQ ID NO: 9.

In certain embodiments, the second antigen-binding protein comprises an antibody heavy chain, the antibody heavy chain comprising the amino acid sequence set forth in SEQ ID NO: 28.

In certain embodiments, the second antigen-binding protein comprises at least one CDR in an antibody light chain variable region VL, the VL comprising the amino acid sequence set forth in SEQ ID NO: 35.

In certain embodiments, the second antigen-binding protein comprises LCDR3, the LCDR3 comprising the amino acid sequence set forth in SEQ ID NO: 29.

In certain embodiments, the second antigen-binding protein comprises LCDR2, the LCDR2 comprising the amino acid sequence set forth in SEQ ID NO: 30 (LAS).

In certain embodiments, the second antigen-binding protein comprises LCDR1, the LCDR1 comprising the amino acid sequence set forth in SEQ ID NO: 31.

In certain embodiments, the second antigen-binding protein comprises a VL, the VL comprising the amino acid sequence set forth in SEQ ID NO: 35.

In certain embodiments, the second antigen-binding protein comprises an antibody light chain constant region.

In certain embodiments, the antibody light chain constant region is derived from a human Igκ constant region.

In certain embodiments, the antibody light chain constant region comprises the amino acid sequence set forth in SEQ ID NO: 19.

In certain embodiments, the second antigen-binding protein comprises an antibody light chain, the antibody light chain comprising the amino acid sequence set forth in SEQ ID NO: 36.

In certain embodiments, the first antigen-binding protein and the second antigen-binding protein in the pharmaceutical combination are in a mass ratio of about 1:5 to 5:1.

In certain embodiments, the first antigen-binding protein and the second antigen-binding protein in the pharmaceutical combination are in a mass ratio of about 1:5.

In certain embodiments, the first antigen-binding protein and the second antigen-binding protein in the pharmaceutical combination are in a mass ratio of about 1:2.5.

In certain embodiments, the first antigen-binding protein and the second antigen-binding protein in the pharmaceutical combination are in a mass ratio of about 1:1.

In certain embodiments, the first antigen-binding protein and the second antigen-binding protein in the pharmaceutical combination are in a mass ratio of about 2.5:1.

In certain embodiments, the first antigen-binding protein and the second antigen-binding protein in the pharmaceutical combination are in a mass ratio of about 5:1.

In another aspect, the present application also provides a pharmaceutical composition comprising the pharmaceutical combination, and optionally one or more pharmaceutically acceptable carriers.

In certain embodiments, the pharmaceutical combination is at a concentration of about 3-300 mg/mL.

In certain embodiments, the pharmaceutical combination is at a concentration of about 3-150 mg/mL.

In certain embodiments, the pharmaceutical combination is at a concentration of about 5-100 mg/mL.

In certain embodiments, the pharmaceutical combination is at a concentration of about 10-100 mg/mL.

In certain embodiments, the pharmaceutical combination is at a concentration of about 10-50 mg/mL.

In certain embodiments, the pharmaceutical combination is at a concentration of 30±5 mg/mL.

In certain embodiments, the pharmaceutical combination is at a concentration of 30±3 mg/mL.

In certain embodiments, the pharmaceutically acceptable carrier comprises a buffer.

In certain embodiments, the buffer comprises an acidic buffer.

In certain embodiments, the buffer comprises a neutral buffer.

In certain embodiments, the buffer is selected from the group consisting of: an acetate-acetic acid buffer, a histidine-histidine hydrochloride buffer, a citrate-citric acid buffer, a phosphate buffer, a histidine-acetic acid buffer, a tris(hydroxymethyl)aminomethane (Tris)-hydrochloride buffer, phosphoric acid-citrate, and a combination thereof.

In certain embodiments, the buffer is selected from the group consisting of: acetate-acetic acid, citrate-citric acid, histidine-histidine hydrochloride, phosphate, Tris-hydrochloride, histidine-acetic acid, phosphoric acid-citrate, and a combination thereof.

In certain embodiments, the buffer is selected from the group consisting of: sodium acetate-acetic acid, sodium citrate-citric acid, histidine-histidine hydrochloride, sodium dihydrogen phosphate-disodium hydrogen phosphate, Tris-hydrochloride, histidine-acetic acid, and phosphoric acid-sodium citrate.

In certain embodiments, the buffer is at a concentration of about 5-100 mM.

In certain embodiments, the buffer is at a concentration of about 5-60 mM.

In certain embodiments, the buffer is at a concentration of about 5-50 mM.

In certain embodiments, the buffer is at a concentration of about 10-40 mM.

In certain embodiments, the buffer is at a concentration of 20±5 mM.

In certain embodiments, the buffer is at a concentration of 20±2 mM.

In certain embodiments, the pharmaceutical composition has a pH value of about 5-7.

In certain embodiments, the pharmaceutical composition has a pH value of about 6±0.5.

In certain embodiments, the pharmaceutical composition has a pH value of about 6±0.3.

In certain embodiments, the pharmaceutical composition has a pH value of about 6±0.1.

In certain embodiments, the pharmaceutical composition has a pH value of about 6.5±0.3.

In certain embodiments, the pharmaceutical composition has a pH value of about 7±0.3.

In certain embodiments, the pharmaceutically acceptable carrier comprises a stabilizer.

In certain embodiments, the stabilizer is selected from the group consisting of: sodium chloride, an amino acid, a sugar alcohol, and a combination thereof.

In certain embodiments, the amino acid is selected from the group consisting of: proline, arginine, glycine, histidine, lysine, methionine, and a combination thereof.

In certain embodiments, the sugar alcohol is selected from the group consisting of: sucrose, mannitol, trehalose, sorbitol, and a combination thereof.

In certain embodiments, the stabilizer is selected from the group consisting of: sucrose, trehalose, sorbitol, mannitol, proline/sucrose, methionine/trehalose, glycine/mannitol, sodium chloride/sucrose, arginine/sucrose, and lysine/trehalose.

In certain embodiments, the content of the stabilizer is about 0.15 wt.%-20 wt.%.

In certain embodiments, the content of the stabilizer is about 0.15 wt.%-10 wt.%.

In certain embodiments, the content of the stabilizer is about 0.15 wt.%-8 wt.%.

In certain embodiments, the content of the stabilizer is about 4±1 wt.% or 8±1 wt.%.

In certain embodiments, the content of the stabilizer is about 4±0.5 wt.% or 8±0.5 wt.%.

In certain embodiments, the content of the stabilizer is about 4 wt.%, 5 wt.%, 7 wt.%, or 8 wt.%.

In certain embodiments, the content of the stabilizer is about 8 wt.%.

In certain embodiments, the pharmaceutically acceptable carrier comprises a surfactant.

In certain embodiments, the surfactant is selected from the group consisting of: a polyoxyethylene sorbitan fatty acid ester, polyethoxylated hydrogenated castor oil, a glycerol fatty acid ester, polysorbate, poloxamer, and a combination thereof.

In certain embodiments, the polysorbate is selected from the group consisting of: polysorbate 20 (PS-20, Tween-20), polysorbate 40 (PS-40), polysorbate 60 (PS-60), and polysorbate 80 (PS-80).

In certain embodiments, the surfactant is selected from the group consisting of: PS-20 (Tween-20) and PS-80 (Tween-80).

In certain embodiments, the surfactant is at a concentration of about 0.001%-0.5%.

In certain embodiments, the surfactant is at a concentration of about 0.005%-0.1%.

In certain embodiments, the surfactant is at a concentration of about 0.005%, 0.01%, 0.03%, 0.05%, or 0.1%.

In certain embodiments, the surfactant is at a concentration of about 0.01%.

In certain embodiments, the surfactant is at a concentration of about 0.03%.

In certain embodiments, the pharmaceutically acceptable carrier is selected from the group consisting of: (1) 20 mM histidine-acetic acid, 8 wt.% sucrose, and 0.03 wt.% Tween-80; (2) 20 mM sodium acetate-acetic acid, 8 wt.% trehalose, and 0.03 wt.% Tween-80; (3) 20 mM histidine-acetic acid, 1.5 wt.% proline, 5 wt.% sucrose, and 0.03 wt.% Tween-80; (4) 20 mM sodium acetate-acetic acid, 0.15 wt.% methionine, 8 wt.% trehalose, and 0.03 wt.% Tween-80; (5) 20 mM histidine-acetic acid, 8 wt.% sucrose, and 0.01 wt.% Tween-80; (6) 20 mM sodium acetate-acetic acid, 8 wt.% trehalose, and 0.01 wt.% Tween-80; (7) 20 mM histidine-acetic acid, 1.5 wt.% proline, 5 wt.% sucrose, and 0.01 wt.% Tween-80; and (8) 20 mM sodium acetate-acetic acid, 0.15 wt.% methionine, 8 wt.% trehalose, and 0.01 wt.% Tween-80.

In another aspect, the present application provides use of the pharmaceutical combination and/or the pharmaceutical composition in the preparation of a medicament for preventing and/or treating diseases and/or disorders.

In certain embodiments, the disease and/or condition includes a CD73-mediated disease and/or disorder.

In certain embodiments, the diseases and/or disorders includes a tumor.

In certain embodiments, the tumor includes a solid tumor and/or a hematological tumor.

In certain embodiments, the diseases and/or disorders are selected from the group consisting of: pancreatic cancer, breast cancer, and idiopathic pulmonary fibrosis (IPF).

In certain embodiments, the breast cancer includes triple-negative breast cancer.

In another aspect, the present application also provides use of the pharmaceutical combination and/or the pharmaceutical composition in the preparation of a detection kit for detecting the presence and/or content of CD73 protein.

Other aspects and advantages of the present application will be readily apparent to those skilled in the art from the following detailed description. Only exemplary embodiments of the present application have been shown and described in the following detailed description. As those skilled in the art will recognize, the content of the present application enables those skilled in the art to make changes in the specific embodiments disclosed without departing from the spirit and scope of the invention to which the present application relates. Accordingly, the drawings and description of the present application are provided only for purpose of illustration rather than limitation.

### BRIEF DESCRIPTION OF THE DRAWINGS

The specific features of the invention to which the present application relates are set forth in the appended claims. The features and advantages of the invention to which the present application relates may be more fully understood with reference to the exemplary embodiments and drawings described in detail below. The drawings are briefly described as follows:
FIG. 1 shows the effect of the cocktail antibody therapy of the present application on inhibiting the enzymatic activity of CD73.
FIG. 2 shows the binding affinity curves and response values of 900698 and 900725 to human CD73 protein under different loading conditions.
FIG. 3 shows the anti-tumor effect of the anti-CD73 cocktail antibody in a pancreatic cancer animal model.
FIG. 4 shows the anti-tumor effect of the anti-CD73 cocktail antibody in a triple-negative breast cancer animal model.
FIG. 5 shows a schematic view of an administration regimen for an idiopathic pulmonary fibrosis (IPF) mouse model.
FIG. 6 shows the body weight of mice in each group of the idiopathic pulmonary fibrosis (IPF) mouse model.
FIG. 7 shows the detection results of airway hyperresponsiveness (area under the curve of Penh change rate-Mch concentration) in mice.
FIG. 8 is categorical bar charts showing the counting results of the total number of inflammation-related cells and the number of lymphocytes in the bronchoalveolar lavage fluid.
FIG. 9 shows the pathological score results of idiopathic pulmonary fibrosis.
FIG. 10 shows representative pictures of pathological sections of lung tissue according to the pathological score results in the experiment.

### Examples

The implementation of the invention to which the present application relates is described below with reference to specific embodiments, and other advantages and effects of the invention to which the present application relates will be readily apparent to those skilled in the art from the disclosure of the present specification.

### Definitions of Terms

In the present application, the term "pharmaceutical combination" generally refers to a combination comprising at least two active ingredients/therapeutic agents. In some embodiments, each active ingredient/therapeutic agent may be prepared as a separate formulation (a solid, liquid, gel, etc.); in some embodiments, active ingredients/therapeutic agents may be present in different containers, and may be formulated into desired formulations, simultaneously or separately, with suitable carriers, as desired; in some embodiments, active ingredients/therapeutic agents may be derived from different sources; in some embodiments, active ingredients/therapeutic agents may be present in the form of a mixture.

In the present application, the term "CD73" is also referred to as "NT5E", "cluster of differentiation 73", "5'-nucleotidase (5'-NT)", or "extracellular 5'-nucleotidase". The term encompasses "full-length" unprocessed CD73, as well as any form of CD73 that results from cellular processing. In the present application, the term "CD73" includes full-length wild-type CD73 and mutants, fragments, variants, isotypes, and homologs thereof. In certain embodiments, the CD73 may include human CD73. For example, the sequence for human CD73 can be found under the Uniprot accession number P21589.

In the present application, the term "antigen-binding protein" generally refers to a protein having an antigen-binding ability obtained by artificial means from a natural state. The "antigen-binding protein" may comprise an antigen-binding moiety, and optionally, a framework or scaffold moiety that allows the antigen-binding moiety to adopt a conformation that facilitates binding of the antigen-binding moiety to an antigen. The antigen-binding protein may comprise, for example, antibody-derived protein framework regions (FRs) or alternative protein framework regions or artificial framework regions with grafted CDRs or CDR derivatives. Such frameworks include, but are not limited to, antibody-derived framework regions comprising mutations introduced, for example, to stabilize the three-dimensional structure of the antigen-binding protein, as well as fully synthetic framework regions comprising, for example, biocompatible polymers. See, e.g., Korndorfer et al., 2003, Proteins: Structure, Function, and Bioinformatics, 53(1): 121-129 (2003); and Roque et al., Biotechnol. Prog. 20: 639-654 (2004). Examples of the antigen-binding protein include, but are not limited to: human antibodies or humanized antibodies, chimeric antibodies, recombinant antibodies, single-chain antibodies, bifunctional antibodies, trifunctional antibodies, tetrafunctional antibodies, Fab, Fab', Fv fragments, F(ab')₂, F(ab)₂, scFv, di-scFv, dAb, IgD antibodies, IgE antibodies, IgM antibodies, IgG1 antibodies, IgG2 antibodies, IgG3 antibodies, or IgG4 antibodies, and fragments thereof.

In the present application, the terms "variable domain" and "variable region" are used interchangeably and generally refer to a portion of an antibody heavy and/or light chain. The variable domains of the heavy and light chains can be referred to as "V_{H}" and "V_{L}", respectively (or "VH" and "VL", respectively). These domains are generally the most variable portions of an antibody (relative to other antibodies of the same type) and comprise antigen-binding sites.

In the present application, the term "variable" generally means that certain segments of the variable domains may differ greatly in sequence among antibodies. The variable domain mediates antigen binding and defines the specificity of a specific antibody for its specific antigen. However, the variability is not evenly distributed throughout the entire variable domain. It is generally concentrated in three segments called hypervariable regions (CDRs or HVRs) in the light and heavy chain variable domains. The more highly conserved portions of the variable domains are referred to as framework regions (FRs). The variable domains of natural heavy and light chains each comprise four FRs largely in a β-sheet configuration. The FRs are connected by three CDRs to form a loop connection, and in some cases to form part of a β-sheet structure. The CDRs in each chain are held closely together by the FRs, and, together with the CDRs from the other chain, contribute to the formation of the antigen-binding sites of the antibody (see Kabat et al., Sequences of Immunological Interest, Fifth Edition, National Institute of Health, Bethesda, Md. (1991)).

In the present application, the term "antibody" generally refers to an immunoglobulin or a fragment thereof or a derivative thereof, and encompasses any polypeptide that comprises an antigen-binding site, regardless of whether it is produced *in vitro* or *in vivo.* The term includes, but is not limited to, polyclonal, monoclonal, monospecific, multispecific, nonspecific, humanized, single-chain, chimeric, synthetic, recombinant, hybrid, mutated, and grafted antibodies. Unless otherwise modified by the term "intact", as in "intact antibody", for the purposes of the present disclosure, the term "antibody" also includes antibody fragments such as Fab, F(ab')₂, Fv, scFv, Fd, dAb, and other antibody fragments that retain antigen-binding functions (e.g., specifically binding to CLDN18.2). Generally, such fragments should include an antigen-binding domain. A basic 4-chain antibody unit is a heterotetrameric glycoprotein consisting of two identical light (L) chains and two identical heavy (H) chains. An IgM antibody consists of 5 basic heterotetrameric units along with an additional polypeptide called the J chain, and contains 10 antigen-binding sites. In contrast, an IgA antibody comprises 2-5 basic 4-chain units that can polymerize with the J chain to form multivalent combinations. For IgG, the 4-chain unit typically has a molecular weight of about 150,000 daltons. Each L chain is linked to an H chain by one covalent disulfide bond, while the two H chains are linked to each other by one or more disulfide bonds depending on the H chain isotype. Each H and L chain also has regularly spaced intrachain disulfide bonds. Each H chain has a variable domain (VH) at the N-terminus, followed by three (for each of the α and γ chains) constant domains (CH) or four (for the µ and ε isotypes) CH domains. Each L chain has a variable domain (VL) at the N-terminus and a constant domain at its other end. VL corresponds to VH, and CL corresponds to the first constant domain of the heavy chain (CH1). Specific amino acid residues are considered to form an interface between the light and heavy chain variable domains. VH and VL pair together to form a single antigen-binding site. For the structures and properties of different classes of antibodies, see, e.g., Basic and Clinical Immunology, 8th Edition, Daniel P. Sties, Abba I. Terr and Tristram G. Parsolw (eds), Appleton & Lange, Norwalk, Conn., 1994, page 71 and chapter 6. The L chains from any vertebrate species can be divided into one of two distinctly different types, κ and λ, based on the amino acid sequences of constant domains thereof. Immunoglobulins can be divided into different classes or isotypes, based on the amino acid sequences of the constant domains of the heavy chains (CH). There are currently five classes of immunoglobulins: IgA, IgD, IgE, IgG, and IgM, which have heavy chains designated α, δ, ε, γ, and µ, respectively. Based on the relatively small differences in CH sequence and function, the γ and α classes are further divided into subclasses, e.g., humans express the following subclasses: IgG1, IgG2A, IgG2B, IgG3, IgG4, IgA1, and IgK1.

In the present application, the term "CDR", also known as "complementarity-determining region", generally refers to a region in an antibody variable domain, which is highly variable in sequence and/or forms a structurally defined loop. For example, an antibody may comprise six CDRs; three in the VH (HCDR1, HCDR2, and HCDR3) and three in the VL (LCDR1, LCDR2, and LCDR3). In certain embodiments, a naturally occurring camelid antibody consisting of only heavy chains is also capable of functioning normally and stably in the absence of light chains. See, e.g., Hamers-Casterman et al., Nature 363: 446-448 (1993); Sheriff et al., Nature Struct. Biol. 3: 733-736 (1996). In the present application, the term "FR" generally refers to a more highly conserved portion of an antibody variable domain, which is referred to as a framework region. Generally, the variable domains of natural heavy and/or light chains each comprise four FRs, namely four in the VH (H-FR1, H-FR2, H-FR3, and H-FR4), and/or four in the VL (L-FR1, L-FR2, L-FR3, and L-FR4).

In the present application, the term "antigen-binding fragment" generally refers to one or more fragments having the ability to specifically bind to an antigen. In the present application, the antigen-binding fragment may include a Fab, a Fab', an F(ab)₂, an Fv fragment, an F(ab')₂, an scFv, a di-scFv, and/or a dAb.

In the present application, the term "Fab" generally refers to an antigen-binding fragment of an antibody. As described above, an intact antibody may be digested with papain. The antibody is digested with papain to produce two identical antigen-binding fragments, namely "Fab" fragments, and a residual "Fc" fragment (i.e., the Fc region, supra). The Fab fragment may consist of one intact L chain and the variable region of one heavy chain along with the first constant region (C_{H}1) of that H chain (V_{H}).

In the present application, the term "Fab' fragment" generally refers to a monovalent antigen-binding fragment of a human monoclonal antibody, which is slightly larger than the Fab fragment. For example, the Fab' fragment may include the entire light chain, the entire heavy chain variable region, and all or part of the first and second constant regions of the heavy chain. For example, the Fab' fragment may also include part or all of the 220-330 amino acid residues of the heavy chain.

In the present application, the term "F(ab')2" generally refers to an antibody fragment produced by pepsin digestion of an intact antibody. The F(ab')2 fragment contains two Fab fragments held together by disulfide bonds and part of a hinge region. The F(ab')2 fragment has divalent antigen-binding activity and is capable of cross-linking antigens.

In the present application, the term "Fv fragment" generally refers to a monovalent antigen-binding fragment of a human monoclonal antibody, which comprises all or part of the heavy chain variable region and the light chain variable region, and lacks the heavy chain constant region and the light chain constant region. The heavy chain variable region and the light chain variable region include, for example, CDRs. For example, an Fv fragment comprises all or part of the amino-terminal variable regions of about 110 amino acids in the heavy and light chains.

In the present application, the term "scFv" generally refers to a fusion protein comprising at least one antibody fragment containing a light chain variable region and at least one antibody fragment containing a heavy chain variable region, wherein the light and heavy chain variable regions are contiguously linked (e.g., via a synthetic linker such as a short flexible polypeptide linker), and are capable of being expressed as a single-chain polypeptide, with the scFv retaining the specificity of the intact antibody from which it is derived. Unless otherwise specified, the scFv, as used herein, may have the VL and VH variable regions in any order (e.g., relative to the N-terminus and C-terminus of the polypeptide), and may comprise VL-linker-VH or VH-linker-VL.

In the present application, the term "dAb" generally refers to an antigen-binding fragment having a VH domain and a VL domain, or having a VH domain or a VL domain. See, e.g., Ward et al. (Nature, 1989 Oct 12; 341(6242): 544-6); Holt et al., Trends Biotechnol., 2003, 21(11): 484-490; and WO 06/030220, WO 06/003388, and other published patent applications by Domantis Ltd.

In the present application, the term "monoclonal antibody" generally refers to an antibody molecule preparation of a single-molecule composition. Monoclonal antibodies generally have high specificity for a single antigenic site. Moreover, unlike conventional polyclonal antibody formulations (which typically have different antibodies directed against different determinants), each monoclonal antibody is directed against a single determinant on the antigen. In addition to their specificity, the monoclonal antibodies are advantageous in that they can be synthesized by hybridoma culture without contamination by other immunoglobulins. The modifier "monoclonal" indicates the characteristic of an antibody obtained from a population of substantially homogeneous antibodies, and is not to be construed as requiring production of the antibody by any particular method. For example, the monoclonal antibodies used in the present application may be prepared in hybridoma cells, or may be prepared by the recombinant DNA method.

In the present application, the term "chimeric antibody" generally refers to an antibody in which the variable region is derived from one species and the constant region is derived from another species. Generally, the variable region is derived from an antibody ("parent antibody") of a laboratory animal such as a rodent, and the constant region is derived from a human antibody, such that the resulting chimeric antibody is less likely to induce an adverse immune response in a human individual as compared to the parent (e.g., mouse-derived) antibody.

In the present application, the term "humanized antibody" generally refers to an antibody in which some or all of the amino acids outside the CDRs of a non-human antibody (e.g., a mouse antibody) are replaced with corresponding amino acids derived from a human immunoglobulin. In the CDRs, small additions, deletions, insertions, substitutions, or modifications of amino acids may also be permissible, so long as they retain the ability of the antibody to bind to a specific antigen. A humanized antibody may optionally comprise at least a portion of a human immunoglobulin constant region. "Humanized antibody" retains antigen specificity similar to that of the original antibody. "Humanized" forms of non-human (e.g., murine) antibodies are chimeric antibodies minimally comprising sequences derived from non-human immunoglobulins. In certain cases, CDR residues of a human immunoglobulin (recipient antibody) may be replaced with CDR residues from a non-human species (donor antibody) (such as mouse, rat, rabbit, or non-human primate) having the desired properties, affinity, and/or capabilities. In certain cases, FR residues of a human immunoglobulin may be replaced with corresponding non-human residues. Furthermore, humanized antibodies may comprise amino acid modifications that are not present in the recipient antibody or in the donor antibody. These modifications may be made to further improve the properties of the antibodies, such as binding affinity.

The term "fully human antibody" generally refers to an antibody that comprises only human immunoglobulin protein sequences. A fully human antibody may contain mouse glycochains if it is produced in a mouse, in a mouse cell, or in a hybridoma derived from a mouse cell. Similarly, "mouse antibody" or "rat antibody" refers to an antibody that comprises only mouse or rat immunoglobulin sequences, respectively. Fully human antibodies can be generated in the human body or in transgenic animals with human immunoglobulin germline sequences through phage display or other molecular biology methods. Exemplary techniques that can be used to prepare antibodies are described in U.S. Patents: 6,150,584; 6,458,592; and 6,420,140. Other techniques, such as the use of libraries, are known in the art.

In the present application, the term "directly linked" is contrasted with the term "indirectly linked", and the term "directly linked" generally refers to a direct linkage. For example, the direct linkage may be a case where substances are directly linked without a spacer therebetween. The spacer may be a linker. For example, the linker may be a peptide linker. The term "indirectly linked" generally refers to a case where the substances are indirectly linked. For example, the indirect linkage may be a linkage via a spacer. For example, in the isolated antigen-binding protein of the present application, the C-terminus of L-FR1 and the N-terminus of LCDR1 may be linked directly or indirectly.

In the present application, the term "pharmaceutical composition" generally refers to a composition for preventing/treating diseases or disorders. The pharmaceutical composition may comprise one or more active ingredients, and optionally a pharmaceutically acceptable carrier. For example, the pharmaceutical composition may further comprise suitable formulations of one or more (pharmaceutically effective) carriers, stabilizers, excipients, diluents, solubilizers, surfactants, emulsifiers, and/or preservatives. The acceptable ingredients of the composition are preferably non-toxic to the recipient at the dose and concentration used. The pharmaceutical composition of the present disclosure includes, but is not limited to, liquid, frozen, and lyophilized compositions.

In the present application, the term "pharmaceutically acceptable carrier" generally includes pharmaceutically acceptable vectors, excipients, and/or stabilizers that are non-toxic to cells or mammals being exposed thereto at the doses and concentrations used. The physiologically acceptable carrier may include, for example, a buffer, an antioxidant, a low-molecular-weight (less than about 10 residues) polypeptide, a protein, a hydrophilic polymer, an amino acid, a monosaccharide, a disaccharide, other carbohydrates, a chelating agent, a sugar alcohol, a salt-forming counterion such as sodium, and/or a nonionic surfactant.

In the present application, the term "subject" generally refers to a human or non-human animal, including but not limited to cats, dogs, horses, pigs, cows, sheep, rabbits, mice, rats, and monkeys.

In the present application, the term "tumor" generally refers to a neoplasm or solid lesion formed by abnormal cell growth. In the present application, the tumor may be a solid tumor or a non-solid tumor. In the present application, the protein, polypeptide, and/or amino acid sequence involved is also to be understood as including at least the following ranges: variants or homologs having the same or similar function as the protein or polypeptide.

In the present application, the variant may be, for example, a protein or a polypeptide obtained by substitution, deletion, or addition of one or more amino acids in the amino acid sequence of the protein and/or the polypeptide (e.g., an antibody or a fragment thereof that specifically binds to CD73). For example, the functional variants may comprise proteins or polypeptides with amino acid changes by substitutions, deletions, and/or insertions of at least 1 (for example, 1-30, 1-20, or 1-10, for another example, 1, 2, 3, 4, or 5) amino acid. The functional variants may substantially retain the biological properties of the protein or the polypeptide before the changes (e.g., substitutions, deletions, or additions). For example, the functional variants may retain at least 60%, 70%, 80%, 90%, or 100% of the biological activity (e.g., antigen-binding ability) of the protein or the polypeptide before the changes. For example, the substitutions may be conservative.

In the present application, the homolog may be a protein or a polypeptide comprising an amino acid sequence having at least about 85% (e.g., at least about 85%, about 90%, about 91%, about 92%, about 93%, about 94%, about 95%, about 96%, about 97%, about 98%, about 99%, or higher) sequence homology to the amino acid sequence of the protein and/or the polypeptide (e.g., an antibody or a fragment thereof that specifically binds to CD73).

In the present application, the homology generally refers to similarity, likeness, or association between two or more sequences. "Percent sequence homology" can be calculated by the following steps: comparing two sequences to be aligned in a comparison window; determining the number of positions at which nucleic acid bases (e.g., A, T, C, G, and I) or amino acid residues (e.g., Ala, Pro, Ser, Thr, Gly, Val, Leu, Ile, Phe, Tyr, Trp, Lys, Arg, His, Asp, Glu, Asn, Gln, Cys, and Met) are identical in the two sequences to give the number of matched positions; dividing the number of matched positions by the total number of positions in the comparison window (i.e., the window size); and multiplying the result by 100 to give a percent sequence homology. Alignment for determining the percent sequence homology can be achieved in a variety of ways known in the art, for example, using publicly available computer software such as BLAST, BLAST-2, ALIGN, or Megalign (DNASTAR) software. Those skilled in the art can determine suitable parameters for alignment of the sequences, including any algorithm necessary to yield optimal alignment within a full-length sequence range or target sequence region being compared. The homology can also be determined by the following methods: FASTA and BLAST. For the description of the FASTA algorithm, see W. R. Pearson and D. J. Lipman, "Improved Tools for Biological Sequence Comparison" (Proc. Natl. Acad. Sci.) 85: 2444-2448, 1988; and D. J. Lipman and W. R. Pearson, "Rapid and Sensitive Protein Similarity Searches", Science, 227: 1435-1441, 1989. For description of the BLAST algorithm, see S. Altschul, W. Gish, W. Miller, E. W. Myers and D. Lipman, "A Basic Local Alignment Search Tool", Journal of Molecular Biology, 215: 403-410, 1990.

In the present application, the term "comprise" or "comprising" generally means including, summarizing, containing, or encompassing. In certain cases, the term also means "being" or "consisting of...".

In the present application, the term "about" generally means varying by 0.5%-10% above or below the stated value, for example, varying by 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below the stated value.

### DETAILED DESCRIPTION OF THE INVENTION

In one aspect, the present application provides a pharmaceutical combination comprising a first antigen-binding protein that specifically binds to CD73, and a second antigen-binding protein that specifically binds to CD73.

In the present application, the first antigen-binding protein has one or more of the following properties:
(1) being capable of specifically binding to CD73 or a functionally active fragment thereof;
(2) being capable of inhibiting enzymatic activity of CD73;
(3) being capable of inducing endocytosis of CD73 on the cell surface; and
(4) being capable of inhibiting the growth and/or proliferation of tumor cells.

In the present application, the first antigen-binding protein and the second antigen-binding protein may have different CD73 antigen-binding epitopes.

In the present application, the first antigen-binding protein and the second antigen-binding protein may bind to the CD73 protein in a non-competitive manner.

In the present application, the first antigen-binding protein and the second antigen-binding protein may be present in the form of a mixture. For example, the first antigen-binding protein and the second antigen-binding protein may be present in the same container and placed in a mixed manner.

In the present application, the first antigen-binding protein may comprise at least one CDR in an antibody heavy chain variable region VH, and the VH may comprise the amino acid sequence set forth in SEQ ID NO: 8.

In the present application, the first antigen-binding protein may comprise HCDR3, and the HCDR3 may comprise the amino acid sequence set forth in SEQ ID NO: 1.

In the present application, the first antigen-binding protein may comprise HCDR2, and the HCDR2 may comprise the amino acid sequence set forth in SEQ ID NO: 2.

In the present application, the first antigen-binding protein may comprise HCDR1, and the HCDR1 may comprise the amino acid sequence set forth in SEQ ID NO: 3.

In the present application, the first antigen-binding protein may comprise HCDR1, HCDR2, and HCDR3, wherein the HCDR1 may comprise the amino acid sequence set forth in SEQ ID NO: 3, the HCDR2 may comprise the amino acid sequence set forth in SEQ ID NO: 2, and the HCDR3 may comprise the amino acid sequence set forth in SEQ ID NO: 1.

In the present application, the first antigen-binding protein may comprise H-FR1, the C-terminus of the H-FR1 is linked directly or indirectly to the N-terminus of the HCDR1, and the H-FR1 may comprise the amino acid sequence set forth in SEQ ID NO: 4.

In the present application, the first antigen-binding protein may comprise H-FR2, the H-FR2 is located between the HCDR1 and the HCDR2, and the H-FR2 may comprise the amino acid sequence set forth in SEQ ID NO: 5.

In the present application, the first antigen-binding protein may comprise H-FR3, the H-FR3 is located between the HCDR2 and the HCDR3, and the H-FR3 may comprise the amino acid sequence set forth in SEQ ID NO: 6.

In the present application, the first antigen-binding protein may comprise H-FR4, the N-terminus of the H-FR4 is linked directly or indirectly to the C-terminus of the H-FR3, and the H-FR4 comprises the amino acid sequence set forth in SEQ ID NO: 7.

In the present application, the first antigen-binding protein may comprise H-FR1, H-FR2, H-FR3, and H-FR4. For example, the H-FR1 comprises the amino acid sequence set forth in SEQ ID NO: 4 or SEQ ID NO: 24, the H-FR2 may comprise the amino acid sequence set forth in SEQ ID NO: 5 or SEQ ID NO: 25, the H-FR3 may comprise the amino acid sequence set forth in SEQ ID NO: 6 or SEQ ID NO: 26, and the H-FR4 may comprise the amino acid sequence set forth in SEQ ID NO: 7.

In the present application, the first antigen-binding protein comprises a VH, and the VH comprises the amino acid sequence set forth in SEQ ID NO: 8.

In the present application, the first antigen-binding protein may comprise an antibody heavy chain constant region.

In the present application, the antibody heavy chain constant region is derived from an IgG heavy chain constant region. For example, the antibody heavy chain constant region is derived from a human IgG heavy chain constant region. For example, the antibody heavy chain constant region is derived from a human IgG1 heavy chain constant region or a human IgG4 heavy chain constant region. For example, the antibody heavy chain constant region comprises the amino acid sequence set forth in SEQ ID NO: 9.

In the present application, the first antigen-binding protein may comprise an antibody heavy chain, and the antibody heavy chain may comprise the amino acid sequence set forth in SEQ ID NO: 10.

In the present application, the first antigen-binding protein may comprise at least one CDR in an antibody light chain variable region VL, and the VL may comprise the amino acid sequence set forth in SEQ ID NO: 18.

In the present application, the first antigen-binding protein may comprise LCDR3, and the LCDR3 may comprise the amino acid sequence set forth in SEQ ID NO: 11.

In the present application, the first antigen-binding protein may comprise LCDR2, and the LCDR2 may comprise the amino acid sequence set forth in SEQ ID NO: 12 (WAS).

In the present application, the first antigen-binding protein may comprise LCDR1, and the LCDR1 may comprise the amino acid sequence set forth in SEQ ID NO: 13.

In the present application, the first antigen-binding protein may comprise LCDR1, LCDR2, and LCDR3, wherein the LCDR1 may comprise the amino acid sequence set forth in SEQ ID NO: 13, the LCDR2 may comprise the amino acid sequence set forth in SEQ ID NO: 12 (WAS), and the LCDR3 may comprise the amino acid sequence set forth in SEQ ID NO: 11.

In the present application, the first antigen-binding protein may comprise L-FR1, the C-terminus of the L-FR1 is linked directly or indirectly to the N-terminus of the LCDR1, and the L-FR1 may comprise the amino acid sequence set forth in SEQ ID NO: 14.

In the present application, the first antigen-binding protein may comprise L-FR2, the L-FR2 is located between the LCDR1 and the LCDR2, and the L-FR2 may comprise the amino acid sequence set forth in SEQ ID NO: 15.

In the present application, the first antigen-binding protein may comprise L-FR3, the L-FR3 is located between the LCDR2 and the LCDR3, and the L-FR3 comprises the amino acid sequence set forth in SEQ ID NO: 16.

In the present application, the first antigen-binding protein may comprise L-FR4, the N-terminus of the L-FR4 is linked directly or indirectly to the C-terminus of the L-FR3, and the L-FR4 comprises the amino acid sequence set forth in SEQ ID NO: 17.

In the present application, the first antigen-binding protein may comprise L-FR1, L-FR2, L-FR3, and L-FR4. For example, the L-FR1 may comprise the amino acid sequence set forth in SEQ ID NO: 14, the L-FR2 may comprise the amino acid sequence set forth in SEQ ID NO: 15, the L-FR3 may comprise the amino acid sequence set forth in SEQ ID NO: 16, and the L-FR4 may comprise the amino acid sequence set forth in SEQ ID NO: 17.

In the present application, the first antigen-binding protein comprises a VL, and the VL comprises the amino acid sequence set forth in SEQ ID NO: 18.

In the present application, the first antigen-binding protein may comprise an antibody light chain constant region.

In the present application, the antibody light chain constant region may be derived from a human Igκ constant region.

In the present application, the antibody light chain constant region may comprise the amino acid sequence set forth in SEQ ID NO: 19.

In the present application, the first antigen-binding protein may comprise an antibody light chain, and the antibody light chain may comprise the amino acid sequence set forth in SEQ ID NO: 20.

In the present application, the first antigen-binding protein may comprise a VH and a VL, wherein the VH may comprise the amino acid sequence set forth in SEQ ID NO: 8, and the VL may comprise the amino acid sequence set forth in SEQ ID NO: 18.

In the present application, the first antigen-binding protein may comprise a heavy chain and a light chain, wherein the heavy chain may comprise the amino acid sequence set forth in SEQ ID NO: 10, and the light chain may comprise the amino acid sequence set forth in SEQ ID NO: 20.

In the present application, the second antigen-binding protein may comprise at least one CDR in an antibody heavy chain variable region VH, and the VH may comprise the amino acid sequence set forth in SEQ ID NO: 27.

In the present application, the second antigen-binding protein may comprise HCDR3, and the HCDR3 may comprise the amino acid sequence set forth in SEQ ID NO: 21.

In the present application, the second antigen-binding protein may comprise HCDR2, and the HCDR2 may comprise the amino acid sequence set forth in SEQ ID NO: 22.

In the present application, the second antigen-binding protein may comprise HCDR1, and the HCDR1 may comprise the amino acid sequence set forth in SEQ ID NO: 23.

In the present application, the second antigen-binding protein may comprise HCDR1, HCDR2, and HCDR3, wherein the HCDR1 may comprise the amino acid sequence set forth in SEQ ID NO: 23, the HCDR2 may comprise the amino acid sequence set forth in SEQ ID NO: 22, and the HCDR3 may comprise the amino acid sequence set forth in SEQ ID NO: 21.

In the present application, the second antigen-binding protein may comprise H-FR1, the C-terminus of the H-FR1 is linked directly or indirectly to the N-terminus of the HCDR1, and the H-FR1 may comprise the amino acid sequence set forth in SEQ ID NO: 24.

In the present application, the second antigen-binding protein may comprise H-FR2, the H-FR2 is located between the HCDR1 and the HCDR2, and the H-FR2 may comprise the amino acid sequence set forth in SEQ ID NO: 25.

In the present application, the second antigen-binding protein may comprise H-FR3, the H-FR3 is located between the HCDR2 and the HCDR3, and the H-FR3 may comprise the amino acid sequence set forth in SEQ ID NO: 26.

In the present application, the second antigen-binding protein may comprise H-FR4, the N-terminus of the H-FR4 is linked directly or indirectly to the C-terminus of the H-FR3, and the H-FR4 comprises the amino acid sequence set forth in SEQ ID NO: 7.

In the present application, the second antigen-binding protein may comprise H-FR1, H-FR2, H-FR3, and H-FR4. For example, the H-FR1 comprises the amino acid sequence set forth in SEQ ID NO: 24, the H-FR2 may comprise the amino acid sequence set forth in SEQ ID NO: 25, the H-FR3 may comprise the amino acid sequence set forth in SEQ ID NO: 26, and the H-FR4 may comprise the amino acid sequence set forth in SEQ ID NO: 7.

In the present application, the second antigen-binding protein comprises a VH, and the VH comprises the amino acid sequence set forth in SEQ ID NO: 8.

In the present application, the second antigen-binding protein may comprise an antibody heavy chain constant region.

In the present application, the antibody heavy chain constant region is derived from an IgG heavy chain constant region. For example, the antibody heavy chain constant region is derived from a human IgG heavy chain constant region. For example, the antibody heavy chain constant region is derived from a human IgG1 heavy chain constant region or a human IgG4 heavy chain constant region. For example, the antibody heavy chain constant region comprises the amino acid sequence set forth in SEQ ID NO: 9.

In the present application, the second antigen-binding protein may comprise an antibody heavy chain, and the antibody heavy chain may comprise the amino acid sequence set forth in SEQ ID NO: 28. In the present application, the second antigen-binding protein may comprise at least one CDR in an antibody light chain variable region VL, and the VL may comprise the amino acid sequence set forth in SEQ ID NO: 35.

In the present application, the second antigen-binding protein may comprise LCDR3, and the LCDR3 may comprise the amino acid sequence set forth in SEQ ID NO: 29.

In the present application, the second antigen-binding protein may comprise LCDR2, and the LCDR2 may comprise the amino acid sequence set forth in SEQ ID NO: 30 (LAS).

In the present application, the second antigen-binding protein may comprise LCDR1, and the LCDR1 may comprise the amino acid sequence set forth in SEQ ID NO: 31.

In the present application, the second antigen-binding protein may comprise LCDR1, LCDR2, and LCDR3, wherein the LCDR1 may comprise the amino acid sequence set forth in SEQ ID NO: 31, the LCDR2 may comprise the amino acid sequence set forth in SEQ ID NO: 30 (LAS), and the LCDR3 may comprise the amino acid sequence set forth in SEQ ID NO: 29.

In the present application, the second antigen-binding protein may comprise L-FR1, the C-terminus of the L-FR1 is linked directly or indirectly to the N-terminus of the LCDR1, and the L-FR1 may comprise the amino acid sequence set forth in SEQ ID NO: 32.

In the present application, the second antigen-binding protein may comprise L-FR2, the L-FR2 is located between the LCDR1 and the LCDR2, and the L-FR2 may comprise the amino acid sequence set forth in SEQ ID NO: 33.

In the present application, the second antigen-binding protein may comprise L-FR3, the L-FR3 is located between the LCDR2 and the LCDR3, and the L-FR3 comprises the amino acid sequence set forth in SEQ ID NO: 34.

In the present application, the second antigen-binding protein may comprise L-FR4, the N-terminus of the L-FR4 is linked directly or indirectly to the C-terminus of the L-FR3, and the L-FR4 comprises the amino acid sequence set forth in SEQ ID NO: 17.

In the present application, the second antigen-binding protein may comprise L-FR1, L-FR2, L-FR3, and L-FR4. For example, the L-FR1 may comprise the amino acid sequence set forth in SEQ ID NO: 32, the L-FR2 may comprise the amino acid sequence set forth in SEQ ID NO: 33, the L-FR3 may comprise the amino acid sequence set forth in SEQ ID NO: 34, and the L-FR4 may comprise the amino acid sequence set forth in SEQ ID NO: 17.

In the present application, the second antigen-binding protein comprises a VL, and the VL comprises the amino acid sequence set forth in SEQ ID NO: 35.

In the present application, the second antigen-binding protein may comprise an antibody light chain constant region.

In the present application, the antibody light chain constant region may be derived from a human Igκ constant region.

In the present application, the antibody light chain constant region may comprise the amino acid sequence set forth in SEQ ID NO: 19.

In the present application, the second antigen-binding protein may comprise an antibody light chain, and the antibody light chain may comprise the amino acid sequence set forth in SEQ ID NO: 36.

In the present application, the second antigen-binding protein may comprise a VH and a VL, wherein the VH may comprise the amino acid sequence set forth in SEQ ID NO: 27, and the VL may comprise the amino acid sequence set forth in SEQ ID NO: 35.

In the present application, the second antigen-binding protein may comprise a heavy chain and a light chain, wherein the heavy chain may comprise the amino acid sequence set forth in SEQ ID NO: 28, and the light chain may comprise the amino acid sequence set forth in SEQ ID NO: 36.

In the present application, the first antigen-binding protein and the second antigen-binding protein in the pharmaceutical combination may be mixed in a certain mass ratio, for example, in a mass ratio of about 1:5 to 5:1. For example, the first antigen-binding protein and the second antigen-binding protein are in a mass ratio of about 1:5. For example, the first antigen-binding protein and the second antigen-binding protein are in a mass ratio of about 1:2.5. For example, the first antigen-binding protein and the second antigen-binding protein are in a mass ratio of about 1:1. For example, the first antigen-binding protein and the second antigen-binding protein are in a mass ratio of about 2.5:1. For example, the first antigen-binding protein and the second antigen-binding protein are in a mass ratio of about 5:1.

In the present application, the pharmaceutical combination may be formulated to be administered by intravenous injection.

### Pharmaceutical Composition

In another aspect, the present application also provides a pharmaceutical composition comprising the pharmaceutical combination, and optionally one or more pharmaceutically acceptable carriers.

In the present application, the pharmaceutical composition may be a mixture comprising the first antigen-binding protein and the second antigen-binding protein, which may further comprise an inactive ingredient to be formulated into a pharmaceutically acceptable formulation.

In the present application, the pharmaceutical combination is at a concentration of about 3-300 mg/mL, optionally about 3-150 mg/mL, optionally about 5-100 mg/mL, optionally about 10-100 mg/mL, and optionally about 10-50 mg/mL, e.g., about 30±5 mg/mL, e.g., about 30±3 mg/mL.

In the present application, the pharmaceutical composition may comprise a buffer. For example, the buffer may be an acidic buffer. For example, the buffer may be a neutral buffer.

For example, the buffer may be selected from the group consisting of: an acetate-acetic acid buffer, a histidine-histidine hydrochloride buffer, a citrate-citric acid buffer, a phosphate buffer, a histidine-acetic acid buffer, a tris(hydroxymethyl)aminomethane (Tris)-hydrochloride buffer, phosphoric acid-citrate buffer, and a combination thereof.

For example, the buffer may be selected from the group consisting of: acetate-acetic acid, citrate-citric acid, histidine-histidine hydrochloride, phosphate, Tris-hydrochloride, histidine-acetic acid, phosphoric acid-citrate, and a combination thereof.

For example, the buffer may be selected from one or more of the group consisting of: sodium acetate-acetic acid, sodium citrate-citric acid, histidine-histidine hydrochloride, sodium dihydrogen phosphate-disodium hydrogen phosphate, Tris-hydrochloride, histidine-acetic acid, and phosphoric acid-sodium citrate. For example, the buffer may be a histidine-acetic acid buffer.

In the present application, the buffer may be at a concentration of about 5-100 mM, optionally about 5-60 mM, optionally about 5-50 mM, optionally about 10-40 mM, optionally about 20±5 mM, and optionally about 20±2 mM.

In the present application, the pharmaceutical composition may have a pH of about 5-7. For example, the pharmaceutical composition has a pH value of about 6±0.5. For example, the pharmaceutical composition has a pH value of about 6±0.3. For example, the pharmaceutical composition has a pH value of about 6±0.1. For example, the pharmaceutical composition has a pH value of about 6.5±0.3. For example, the pharmaceutical composition has a pH value of about 7±0.3.

In the present application, the pharmaceutical composition may comprise a stabilizer. In the present application, the stabilizer may be selected from the group consisting of: sodium chloride, an amino acid, a sugar alcohol, and a combination thereof. For example, the amino acid may be selected from the group consisting of: proline, arginine, glycine, histidine, lysine, methionine, and a combination thereof. For example, the sugar alcohol may be selected from the group consisting of: sucrose, mannitol, trehalose, sorbitol, and a combination thereof.

For example, the stabilizer may be selected from one or more of the group consisting of: sucrose, trehalose, sorbitol, mannitol, proline/sucrose, methionine/trehalose, glycine/mannitol, sodium chloride/sucrose, arginine/sucrose, and lysine/trehalose. For example, the stabilizer of the pharmaceutical composition may be sucrose.

In the present application, the content of the stabilizer in the pharmaceutical composition is about 0.15 wt.%-20 wt.%; optionally, the content of the stabilizer is about 0.15 wt.%-10 wt.%; optionally, the content of the stabilizer is about 0.15 wt.%-8 wt.%; optionally, the content of the stabilizer is about 4±1 wt.% or 8±1 wt.%; optionally, the content of the stabilizer is about 4±0.5 wt.% or 8±0.5 wt.%; and optionally, the content of the stabilizer is about 4 wt.%, 5 wt.%, 7 wt.%, or 8 wt.%. For example, the content of the stabilizer is about 0.15 wt.%, about 0.2 wt.%, about 0.3 wt.%, about 0.4 wt.%, 0.5 wt.%, about 0.6 wt.%, about 0.8 wt.%, about 1 wt.%, about 1.5 wt.%, about 2 wt.%, about 2.5 wt.%, about 3 wt.%, about 3.5 wt.%, about 4 wt.%, about 4.5 wt.%, about 5 wt.%, about 5.5 wt.%, about 6 wt.%, about 7 wt.%, or about 8 wt.%.

In the present application, the pharmaceutical composition may comprise a surfactant.

In the present application, the surfactant may be selected from the group consisting of: a polyoxyethylene sorbitan fatty acid ester, polyethoxylated hydrogenated castor oil, a glycerol fatty acid ester, polysorbate, poloxamer, and a combination thereof. In the present application, the polysorbate may be selected from the group consisting of: polysorbate 20 (PS-20, Tween-20), polysorbate 40 (PS-40), polysorbate 60 (PS-60), and polysorbate 80 (PS-80). For example, the surfactant may include Tween-20 (PS-20) and/or Tween-80 (PS-80). For example, the surfactant is at a concentration of about 0.001%-0.5%, optionally about 0.005%-0.1%, optionally about 0.005%, 0.01%, 0.03%, 0.05%, or 0.1%, and optionally about 0.01% or 0.03%. For example, the surfactant is at a concentration of about 0.005%, about 0.01%, 0.03%, about 0.05%, or about 0.1%.

In the present application, the buffer system of the pharmaceutical composition may comprise 20 mM histidine-acetic acid, 8 wt.% sucrose, and 0.03 wt.% Tween-80.

In the present application, the buffer system of the pharmaceutical composition may comprise 20 mM sodium acetate-acetic acid, 8 wt.% trehalose, and 0.03 wt.% Tween-80.

In the present application, the buffer system of the pharmaceutical composition may comprise 20 mM histidine-acetic acid, 1.5 wt.% proline, 5 wt.% sucrose, and 0.03 wt.% Tween-80.

In the present application, the buffer system of the pharmaceutical composition may comprise 20 mM sodium acetate-acetic acid, 0.15 wt.% methionine, 8 wt.% trehalose, and 0.03 wt.% Tween-80.

In the present application, the buffer system of the pharmaceutical composition may comprise 20 mM histidine-acetic acid, 8 wt.% sucrose, and 0.01 wt.% Tween-80.

In the present application, the buffer system of the pharmaceutical composition may comprise 20 mM sodium acetate-acetic acid, 8 wt.% trehalose, and 0.01 wt.% Tween-80.

In the present application, the buffer system of the pharmaceutical composition may comprise 20 mM histidine-acetic acid, 1.5 wt.% proline, 5 wt.% sucrose, and 0.01 wt.% Tween-80.

In the present application, the buffer system of the pharmaceutical composition may comprise 20 mM sodium acetate-acetic acid, 0.15 wt.% methionine, 8 wt.% trehalose, and 0.01 wt.% Tween-80.

### Use

In another aspect, the present application provides use of the pharmaceutical combination and/or the pharmaceutical composition in the preparation of a medicament for preventing and/or treating diseases and/or disorders.

In another aspect, the present application also provides a method for preventing and/or treating diseases and/or disorders, which may comprise administering to a subject in need thereof the pharmaceutical combination and/or the pharmaceutical composition of the present application.

In the present application, the administration may be performed in different ways, such as intravenous, intratumoral, intraperitoneal, subcutaneous, intramuscular, topical, or intradermal administration.

In another aspect, the pharmaceutical combination and/or the pharmaceutical composition of the present application may be used for preventing and/or treating disease and/or disorders.

In the present application, the disease and/or condition may be diseases and/or disorders mediated by abnormal expression of CD73.

In the present application, the diseases and/or disorders may include a tumor.

In the present application, the tumor may include a solid tumor and/or a hematological tumor.

In the present application, the diseases and/or disorders may include pancreatic cancer, breast cancer, and/or idiopathic pulmonary fibrosis (IPF). For example, the breast cancer may include triple-negative breast cancer.

In another aspect, the present application also provides a method for detecting CD73 in a sample, which comprises administering the pharmaceutical combination and/or the pharmaceutical composition.

In the present application, the method for detecting CD73 in a sample may be an *in vitro* method. In the present application, the method for detecting CD73 in a sample may be for non-therapeutic purposes. In the present application, the method for detecting CD73 in a sample is not a diagnostic method.

In another aspect, the present application also provides a reagent or a kit for detecting CD73 in a sample, which comprises the pharmaceutical combination and/or the pharmaceutical composition.

In another aspect, the present application also provides use of the pharmaceutical combination and/or the pharmaceutical composition in the preparation of a kit for detecting the presence and/or content of CD73 in a sample.

Without being bound by any theory, the following examples are intended only to illustrate the pharmaceutical combination, preparation method, use, etc., of the present application, and are not intended to limit the scope of the present application.

### Example

### General determination methods

SEC-UPLC: the purity was determined using an ACQUITY UPLC ^{®} Protein BEH SEC analytical column (4.6 mm × 150 mm) and a Waters ultra-performance liquid chromatograph (Acquity H Class) according to high-performance liquid chromatography of General Chapter 0514 in the *Pharmacopoeia of the People's Republic of China* (2015 edition, volume III) and calculated by an area normalization method.

CEX-HPLC: the purity was determined using a ProPac^{™} WCX-10 analytical column (4 mm × 250 mm) and a Water high-performance liquid chromatograph (E2695) according to high-performance liquid chromatography of the general chapter in the *Pharmacopoeia of the People's Republic of China* (2015 edition, volume III) and calculated by an area normalization method.

Tm value determination (IF, full-spectrum fluorescence): the melting temperatures Tm of different formulas were calculated using Uncle (Unchained Labs) heated in a range of 25 °C to 95 °C at 0.3 °C/min through a full-spectrum fluorescence module.

Tagg value determination (SLS, static light scattering): the initial aggregation temperatures Tagg of antibody molecules of different formulas were calculated using an Uncle instrument heated in a range of 25 °C to 95 °C at 0.3 °C/min through a static light scattering module.

### Example 1. Inhibition Experiment of CD73 Enzymatic Activity by Components 900698 and 900725 in Anti-CD73 Cocktail Antibody

900698 (having the heavy chain sequence set forth in SEQ ID NO: 10 and the light chain sequence set forth in SEQ ID NO: 20) and 900725 (having the heavy chain sequence set forth in SEQ ID NO: 28 and the light chain sequence set forth in SEQ ID NO: 36) were diluted to 30 µg/mL with PBS, and then mixed according to 1:5, 1:2.5, 1:1, 2.5:1, and 5:1, followed by 5-fold dilution to 8 gradients. A suspension of cells expressing human CD73 (CHOK1-huCD73-3F4, Huabo Biopharm) in the logarithmic growth phase was prepared into a cell suspension at 4 × 10⁵ cells/mL with a medium. The cell suspension was then added at 100 µL (about 40000 cells) per well to a 96-well U-bottom plate and centrifuged at 1500 rpm for 5 min, and the supernatant was discarded. Then, the serially diluted antibodies were each added at 100 µL per well to resuspend the cells, and the mixture was mixed uniformly and incubated at 37 °C for 20 min. After the incubation, the mixture was centrifuged at 1500 rpm for 5 min, and the supernatant was discarded. The residue was washed once with PBS at 200 µL/well, resuspended in 180 µM AMP at 100 µL/well, and incubated at 37 °C for 60 min. After the incubation, the mixture was centrifuged at 1500 rpm for 5 min. The supernatant at 50 µL/well was taken and transferred to a 96-well black plate, and then the prepared ATP was added at 50 µL/well. The mixture was mixed uniformly and allowed to react at 37 °C for 15 min. Finally, a CellTiter-Glo^{®} substrate was added to a CellTiter-Glo^{®} buffer. The mixture was uniformly and eqilibrated to room temperature, and then added at 100 µL/well to the wells to be tested. The resulting mixture was allowed to react at room temperature for 10 min and then subjected to full-wavelength detection. The percentage of the enzymatic activity was assessed as follows: the residual CD73 activity was: (CHOK1-huCD73-3F4 cells + Ab + ATP + AMP) - (ATP + AMP)/(CHOK1-huCD73-3F4 cells + ATP + AMP) - (ATP + AMP) × 100.

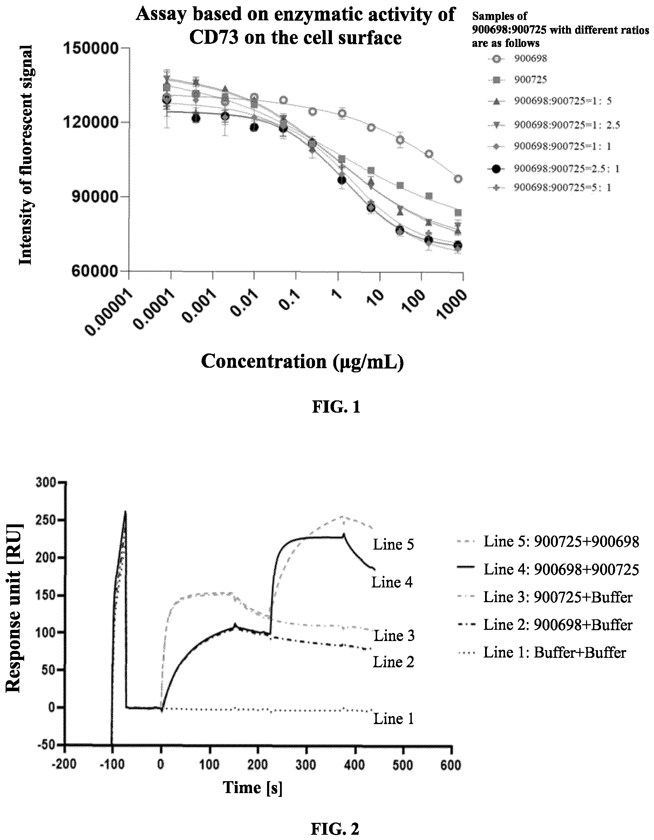

900698, 900725, and mixtures of 900698 and 900725 in different ratios showed inhibitory effects on the enzymatic activity of CD73 expressed on the cell membrane (CHOK1-huCD73-3F4, Huabo Biopharm) as shown in FIG. 1. The results show that when the antibody concentration was 0.1-1 µg/mL, the mixture of 900698 and 900725 had a stronger ability to inhibit the enzymatic activity than that of 900698 and 900725 alone under the same concentration condition (as indicated by the black dashed arrow, the lower plateau value of an enzymatic activity inhibition curve is lower, indicating that the maximum inhibitory ability of the antibody to the enzymatic activity is stronger), which indicates that 900698 and 900725 have a synergistic inhibitory effect after mixing, and the effect of 900698 and 900725 in a ratio of 1:1 seems to be optimal.

### Example 2. SPR Competitive Binding Experiment for Anti-CD73 Cocktail Antibody

To explore whether the antibodies 900698 and 900725 compete for binding to CD73, we performed an SPR competition binding experiment. First, the human CD73 antigen protein (manufacturer: ACRO Biosystems, Catalog No. CD3-H52H7) was captured as a ligand using BIAcore 8k, and then the antibodies were used as analytes. The order for adding the antibody was 900698 + 900725 (900698 was first added, and then 900725 was added) or 900725 + 900698 (900725 was first added, and then 900698 was added), and 900698 + buffer and 900725 + buffer were used as controls.

As shown in Table 1 below and FIG. 2, regardless of whether 900698 or 900725 was added first, the binding of the other antibody to the CD73 antigen was not affected, indicating that the two components 900698 and 900725 of the anti-CD73 cocktail antibody have different binding epitopes on the CD73 antigen and are capable of binding to the CD73 antigen simultaneously.

**Table 1. Assay for binding affinity of 900698 and 900725 for human CD73 protein under different loading conditions**

| Ligand | Analyte 1 | Analyte 2 | ka (1/Ms) | kd (1/s) | KD (M) |
|---|---|---|---|---|---|
| Human CD73 dimer protein | Buffer | 900698 | 1.56E+05 | 1.14E-03 | 7.30E-09 |
| | 900725 | 900698 | 1.35E+05 | 1.70E-03 | 1.26E-08 |
| | Buffer | 900725 | 1.50E+06 | 4.52E-03 | 3.01E-09 |
| | 900698 | 900725 | 1.42E+06 | 5.91E-03 | 4.16E-09 |

### Example 3. Assay for Anti-Tumor Effect of Anti-CD73 Cocktail Antibody in Pancreatic Cancer Animal Model

NPG mice were used in this experiment to establish a BxPC-3 pancreatic cancer animal model for testing the efficacy of the test antibodies. The BxPC-3 human pancreatic cancer cells used in this experiment were incubated in an RPMI-1640 medium supplemented with 10% FBS in an incubator at 37 °C with 5% CO₂. Before continuous cell culturing for ten generations, 100 µL of PBS containing about 1 × 10⁷ BxPC-3 cells was mixed uniformly with an equal volume of Matrigel. The mixture was then inoculated by subcutaneous injection into 36 NPG mice on the right side of the back near the axilla, with an inoculation volume of about 200 µL. The mice were anesthetized with 2%-5% isoflurane prior to the inoculation. On the day of inoculation, 1.0 × 10⁷ PBMCs (100 µL) were injected via the tail vein. When the tumors grew to an average size of about 50-80 mm³, the 36 tumor-bearing mice were randomly divided into 6 groups of 6 based on the tumor volume and body weight. Administration was performed on the day of grouping, and the specific administration regimen is shown in the table below. 900201 was used as a negative control antibody, which does not bind to the CD73 antigen. The amino acid sequence of the heavy chain (HC) of 900201 is set forth in SEQ ID NO: 37, and the amino acid sequence of the light chain (LC) of 900201 is set forth in SEQ ID NO: 38. Oleclumab is an anti-CD73 monoclonal antibody developed by AstraZeneca, currently in Phase 3 clinical trials. Uliledlimab is an anti-CD73 monoclonal antibody developed by I-Mab Biopharma, currently in Phase 1 clinical trials.

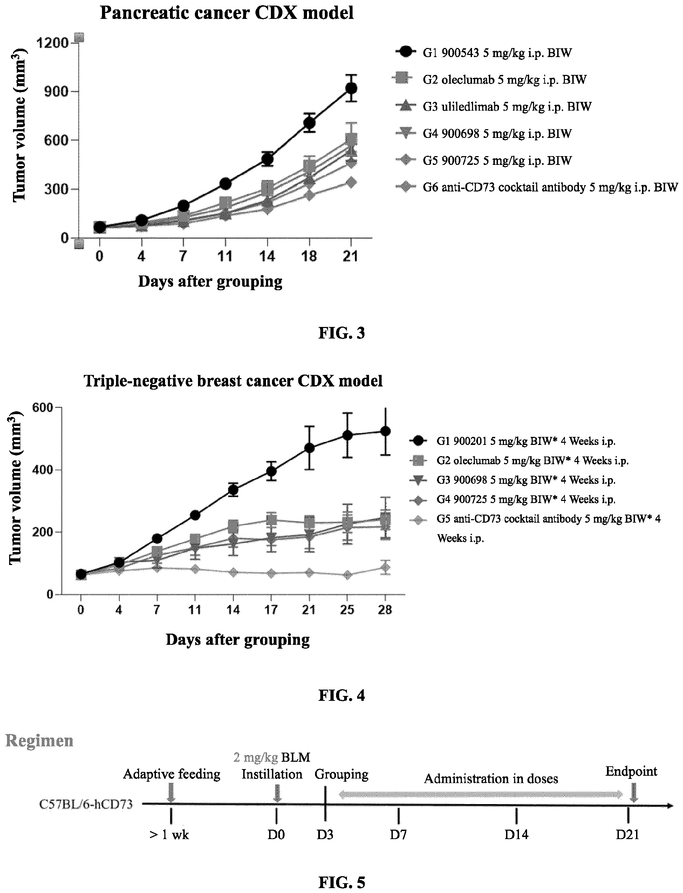

The results are shown in Table 2 below and FIG. 3. Compared with the negative control group 900201, all of the positive drugs oleclumab and uliledlimab, the test drugs 900698 and 900725, as well as the anti-CD73 cocktail antibody could significantly inhibit the growth of BxPC-3 pancreatic cancer, and the anti-CD73 cocktail antibody with the same dose had a better tumor inhibitory effect than those of the positive controls oleclumab and uliledlimab, as well as 900698 and 900725, proving that the anti-CD73 antibody cocktail antibody prepared by mixing 900698 and 900725 in an equal ratio has a very good synergistic anti-tumor effect on pancreatic cancer.

**Table 2. Administration regimen for the pancreatic cancer model**

| Group | Test sample | Number of animals | Administration dose (mg/kg) | Route of administration | Frequency of administration^{a} | Number of doses |
|---|---|---|---|---|---|---|
| G1 | 900201 | 6 | 5 | i.p. | BIW | 6 |
| G2 | oleclumab | 6 | 5 | i.p. | BIW | 6 |
| G3 | uliledlimab | 6 | 5 | i.p. | BIW | 6 |
| G4 | 900698 | 6 | 5 | i.p. | BIW | 6 |
| G5 | 900725 | 6 | 5 | i.p. | BIW | 6 |
| G6 | Anti-CD73 cocktail antibody | 6 | 5 | i.p. | BIW | 8 |

### Example 4. Assay for Anti-Tumor Effect of Anti-CD73 Cocktail Antibody in Triple-Negative Breast Cancer Animal Model

NPG mice were used in this experiment to establish an MDA-MB-231 triple-negative breast cancer animal model for testing the efficacy of the test antibodies. The MDA-MB-231 human breast cancer cells used in this experiment were incubated in an L-15 medium supplemented with 10% FBS in an incubator at 37 °C without CO₂. Before continuous cell culturing for ten generations, about 5.0 × 10⁶ MDA-MB-231 cells were suspended in 100 µL of PBS and then mixed uniformly with an equal volume of Matrigel. The mixture was then inoculated by subcutaneous injection into NPG humanized mice on the right side of the back near the axilla, with an inoculation volume of about 200 µL. The mice were anesthetized with 2%-5% isoflurane prior to the inoculation. On the day of inoculation, 1.0 × 10⁷ PBMCs (100 µL) were injected via the tail vein. When the tumors grew to an average size of about 50-80 mm³, the 30 tumor-bearing mice were randomly divided into 5 groups of 6 based on the tumor volume and body weight. Administration was performed on the day of grouping, and the specific administration regimen is shown in the table below. 900201 was used as a negative control antibody, which does not bind to the CD73 antigen. Oleclumab is an anti-CD73 monoclonal antibody developed by AstraZeneca, currently in Phase 3 clinical trials.

The results are shown in Table 3 below and FIG. 4. Compared with the negative control group 900201, all of the positive drug oleclumab, the test drugs 900698 and 900725, as well as the anti-CD73 cocktail antibody could significantly inhibit the growth of MDA-MB-231 triple-negative breast cancer, and the anti-CD73 cocktail antibody with the same dose had a better tumor inhibitory effect than those of the positive control oleclumab, as well as 900698 and 900725, proving that the anti-CD73 antibody cocktail antibody prepared by mixing 900698 and 900725 in an equal ratio has a very good synergistic anti-tumor effect on triple-negative breast cancer.

**Table 3. Administration regimen for the triple-negative breast cancer model**

| Group | Test sample | Number of animals | Administration dose (mg/kg) | Route of administration | Frequency of administration^{a} | Number of doses |
|---|---|---|---|---|---|---|
| G1 | 900201 | 6 | 5 | i.p. | BIW | 8 |
| G2 | oleclumab | 6 | 5 | i.p. | BIW | 8 |
| G3 | 900698 | 6 | 5 | i.p. | BIW | 8 |
| G4 | 900725 | 6 | 5 | i.p. | BIW | 8 |
| G5 | Anti-CD73 cocktail antibody | 6 | 5 | i.p. | BIW | 8 |

### Example 5. Therapeutic Effect of Anti-CD73 Cocktail Antibody in Idiopathic Pulmonary Fibrosis (IPF) Animal Model

Idiopathic pulmonary fibrosis (IPF) is a fatal disease characterized by progressive and irreversible pulmonary fibrosis, for which no effective treatment method is currently available. Most patients die from progressive respiratory failure within 3-8 years after the onset of symptoms. Although the pathogenesis of IPF is poorly understood, the hallmark pathological features include: inflammatory changes in the lung, hyperproliferation of fibroblasts, and abnormal deposition of the extracellular matrix (ECM). Bleomycin (BLM)-induced fibrosis is widely used in the study of the pathogenesis of idiopathic pulmonary fibrosis.

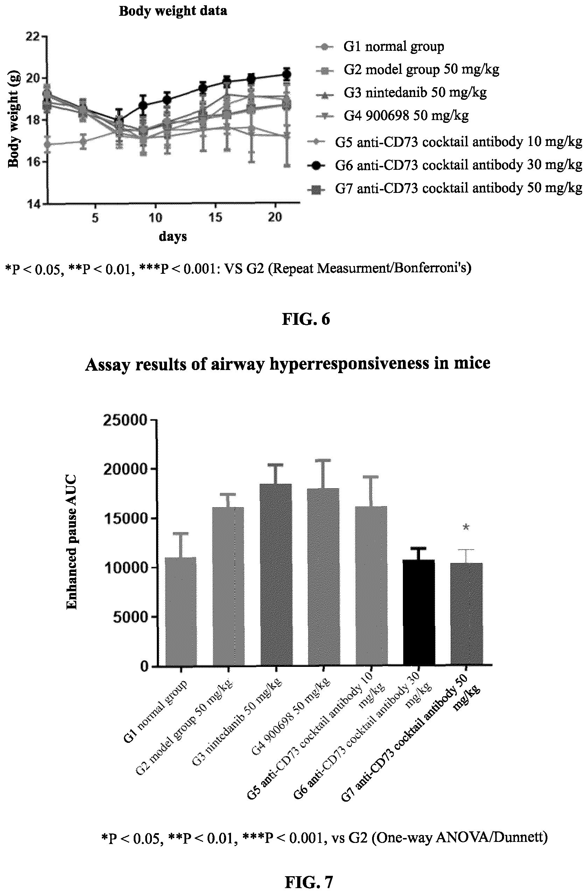

The specific procedures for the experiment involving the bleomycin-induced idiopathic pulmonary fibrosis model in CD73 humanized transgenic mice were as follows: the CD73 humanized mice were grouped according to Table 4 below, i.e., 3 randomly selected animals were directly included in G1 as the normal group, while all other groups of animals received bleomycin by intratracheal instillation for model induction. The specific induction method was as follows: on day 1, the animals were anesthetized by inhalation of 2%-5% isoflurane, and based on the body weight, the animals were intratracheally given bleomycin (2 U/kg, the specific volume of administration was calculated and recorded based on the body weight of the animals). Three days after the instillation of bleomycin, animals with excessive weight loss and slight weight change were removed according to the body weight and state of the animals, and 48 animals were divided into the remaining 6 groups by a BioBook random distribution function, so as to achieve an approximate average weight of each group and reduce the deviation among the groups. All animals were given the drugs from day 4 to the end of the experiment. The specific administration regimen is shown in FIG. 5 and Table 4. Throughout the experiment period, the animals were weighed and recorded three times a week. The body weight results of the mice are shown in FIG. 6. Throughout the experiment period, the body weights of the animals except for the normal group were gradually reduced after modeling, and gradually and continuously increased along with the experiment process and the self-recovery. There was no significant difference among the groups.

**Table 4. Grouping and administration regimen for the idiopathic pulmonary fibrosis (IPF) mouse model**

| **Group** | **Test sample** | **Animal** | **Route of administration** | **Concentration mg/mL** | **Administration concentration** | | **Administration regimen** |
|---|---|---|---|---|---|---|---|
| | | | | | **mg/kg** | **mL/kg** | |
| 1 | Normal group (without receiving bleomycin induction) | 3 | i.p. | / | / | 10 | Starting on the day after grouping, twice a week |
| 2 | Model group (given 900201 negative control antibody) | 8 | i.p. | 5 | 50 | 10 | Starting on the day after grouping, twice a week |
| 3 | Positive drug nintedanib group | 8 | p.o. | 5 | 50 | 10 | Starting on the day after grouping, once a day |
| 4 | 900698 single- drug antibody group | 8 | i.p. | 5 | 50 | 10 | Starting on the day after grouping, twice a week |
| 5 | Anti-CD73 cocktail antibody (900698 + 900725) - low dose | 8 | i.p. | 0.5+0.5 | 5+5 | 10 | Starting on the day after grouping, twice a week |
| 6 | Anti-CD73 cocktail antibody (900698 + 900725) - medium dose | 8 | i.p. | 1.5+1.5 | 15+15 | 10 | Starting on the day after grouping, twice a week |
| 7 | Anti-CD73 cocktail antibody (900698 + 900725) - high dose | 8 | i.p. | 2.5+2.5 | 25+25 | 10 | Starting on the day after grouping, twice a week |

### 5.1 Airway responsiveness assay:

On day 21, all animals received an assay for airway responsiveness to detect the lung function. The airway hyperresponsiveness assay was performed on experimental mice by using a WBP system. First, the mice were given a PBS solution via aerosol inhalation, and then given methacholine (Mch) at concentrations of 1.5625 mg/mL, 3.125 mg/mL, 6.25 mg/mL, 12.5 mg/mL, 25 mg/mL, and 50 mg/mL via continuous aerosol inhalation. The enhanced pause (Penh) at the corresponding concentration was determined, and stimulation was performed at each concentration for 90 s. A curve of the change rate of Penh relative to a baseline-Mch concentration was plotted, and the area under the curve was calculated.

The results are shown in FIG. 7. Compared with the normal animals, the area under the Penh curve (AUC) of the animals in the model group was significantly increased. Compared with the model animal group, the test samples in the medium- and high-dose groups had an improved trend, and the high-dose group was significantly different (P = 0.024 Kruskal-Wallis Test). However, no improvement was observed in the positive control nintedanib group, proving that the anti-CD73 cocktail antibody is superior to the positive control nintedanib in improving the lung function of the mice with idiopathic pulmonary fibrosis.

### 5.2 Determination of the number of inflammatory cells in bronchoalveolar lavage fluid

After completion of the lung function detection, the animals were anesthetized with Zoletil (intraperitoneal injection, 25-50 mg/kg Zoletil, and 5-10 mg/kg xylazine) and then subjected to tracheal intubation. The lungs were first lavaged with 0.4 mL of PBS (containing 1% FBS). Additionally, the lungs were lavaged with 0.4 mL of PBS (containing 1% FBS) for the second and third time. 100 µL of the suspension was taken to count the total number of cells in the bronchoalveolar lavage fluid. The bronchoalveolar lavage fluid was centrifuged at 4 °C at 300 g for 5 min. The cell pellet obtained after the centrifugation was resuspended for smear preparation and stained with the Wright-Giemsa stain to differentiate eosinophils, neutrophils, macrophages, and lymphocytes. The cells were counted under an optical microscope. Experimental data are presented in mean ± standard error (mean ± S.E.M). The data were analyzed using SPSS or Graphpad Prism. The specific analytical methods employed are illustrated in the figure legends and notes below the table. P < 0.05 was considered statistically different.

The cell counting results in the bronchoalveolar lavage fluid show that the animals in the model group had significant climbing relative to the total number of inflammation-related cells and the percentage of lymphocytes in the bronchoalveolar lavage fluid of the normal group; after the positive drug and the anti-CD73 cocktail antibody test samples with different concentrations were given, the levels of the total number of inflammation-related cells and the number of lymphocytes were significantly reduced, indicating that the anti-CD73 cocktail antibody can significantly improve the inflammation degree of idiopathic pulmonary fibrosis.

### 5.3 Pathological fibrosis degree scoring:

After the lavage, the animals were euthanized by cervical dislocation. The left lung was collected, fixed in neutral formalin, embedded in a wax block, and transected. Sections were taken from the upper, middle, and lower segments, respectively, for a total of 3 sections per lung lobe, which were embedded in the wax block. The sections were stained with Masson for pathological fibrosis degree scoring. The scoring criteria are shown in Table 5 below.

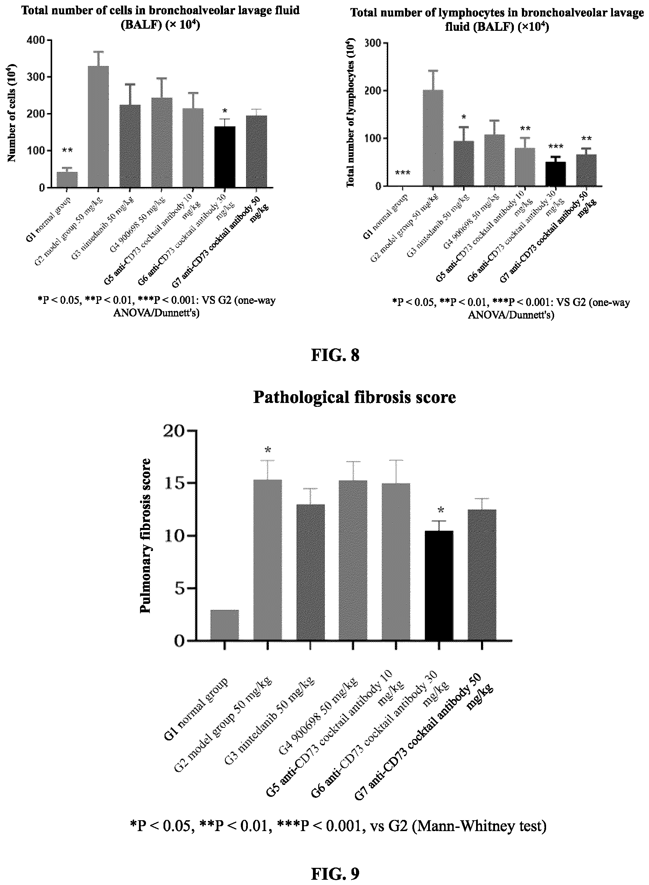

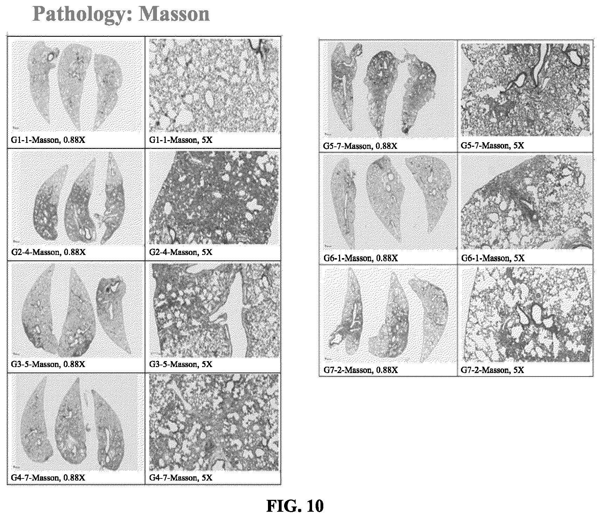

The scoring results are shown in FIGs. 9 and 10. Compared with the normal animals, the animals in the model group showed a significant pulmonary fibrosis change (P < 0.001) after being induced by bleomycin, indicating that the modeling of the bleomycin-induced pulmonary fibrosis model is successful. The positive drug nintedanib group and the anti-CD73 cocktail antibody administration groups all improved the pathological scores. The medium-dose of anti-CD73 cocktail antibody administration group was significantly different from the model group as tested using the Kruskal-Wallis test method, indicating that the anti-CD73 cocktail antibody can effectively improve the pathological score of idiopathic pulmonary fibrosis.

**Table 5. Pathological fibrosis scoring criteria**

| Score | Pathological fibrosis scoring criteria |
|---|---|
| 1 | Normal |
| 3 | Minimal fibrosis thickening |
| 5 | Moderate fibrosis thickening |
| 7 | Fibrosis with lung tissue injury (thick bundle) |
| 8 | Large fibrosis area, showing a "honeycomb shape" |

### Example 6. Comparison of Different Buffer Systems

The antibody was exchanged into each screening buffer (10 mM, as shown in Table 6 below) by using a WaterSep hollow fiber (Discover 12), and the antibody concentration was adjusted to about 5 mg/mL with each buffer. The mixture was sterilized and filtered for later use.

Samples were freshly prepared, and the stability of each buffer was assessed by Tm and Tagg. The results are shown in Tables 6 and 7. At the same time, the freshly prepared samples were subjected to various pressurization conditions, including: high temperature test (40 °C±2 °C, being left to stand for three weeks) and repeated freeze-thaw (5 cycles, ≤ -70 °C/5 °C±3 °C). The stability was assessed by detecting items such as appearance and purity. The results are shown in Tables 8 and 9.

**Table 6. Summary of Tm and Tagg results for 900698 antibody in different buffers**

| Buffer | | Tm (°C) | Tagg (°C) |
|---|---|---|---|
| Type | pH | | |
| Sodium acetate-acetic acid | 6 | 71.9 | 80.4 |
| Sodium citrate-citric acid | 6 | 70.3 | 75.7 |
| Histidine-histidine hydrochloride | 6.5 | 70.6 | 76.4 |
| Sodium dihydrogen phosphate-disodium hydrogen phosphate | 6 | 72.2 | 80.1 |
| Tris-hydrochloride | 7 | 72.9 | 79.5 |
| Histidine-acetic acid | 6 | 69.9 | 77.0 |
| Phosphoric acid-sodium citrate | 6 | 71.3 | 77.4 |

**Table 7. Summary of Tm and Tagg results for 900725 antibody in different buffers**

| Buffer | | Tm (°C) | Tagg (°C) |
|---|---|---|---|
| Type | pH | | |
| Sodium acetate-acetic acid | 6 | 71.8 | 80.4 |
| Sodium citrate-citric acid | 6 | 70.6 | 77.1 |
| Histidine-histidine hydrochloride | 6.5 | 71.3 | 77.0 |
| Sodium dihydrogen phosphate-disodium hydrogen phosphate | 6 | 67.9 | 76.2 |
| Tris-hydrochloride | 7 | 71.7 | 78.2 |
| Histidine-acetic acid | 6 | 70.8 | 76.7 |
| Phosphoric acid-sodium citrate | 6 | 70.7 | 77.5 |

**Table 8. Summary of appearance and purity results for 900698 antibody in different buffer systems under various pressure conditions**

| Buffer | | Freeze-thaw | | High temperature | |
|---|---|---|---|---|---|
| Type | pH | Appearance | SEC purity | Appearance | SEC purity |
| Sodium acetate-acetic acid | 6 | Clarified | 98.8% | Clarified | 96.6% |
| Sodium citrate-citric acid | 6 | Clarified | 99.0% | Clarified | 96.2% |
| Histidine-histidine hydrochloride | 6.5 | Clarified | 98.9% | Clarified | 97.0% |
| Sodium dihydrogen phosphate-disodium hydrogen phosphate | 6 | Clarified | 99.0% | Clarified | 96.0% |
| Tris-hydrochloride | 7 | Clarified | 98.8% | Clarified | 96.6% |
| Histidine-acetic acid | 6 | Clarified | 99.0% | Clarified | 97.0% |
| Phosphoric acid-sodium citrate | 6 | Clarified | 99.0% | Clarified | 96.2% |

**Table 9. Summary of appearance and purity results for 900725 antibody in different buffer systems under various pressure conditions**

| Buffer | | Freeze-thaw | | High temperature | |
|---|---|---|---|---|---|
| Type | pH | Appearance | SEC purity | Appearance | SEC purity |
| Sodium acetate-acetic acid | 6 | Clarified | 98.5% | Clarified | 96.1% |
| Sodium citrate-citric acid | 6 | Clarified | 98.5% | Clarified | 96.0% |
| Histidine-histidine hydrochloride | 6.5 | Clarified | 98.4% | Clarified | 96.5% |
| Sodium dihydrogen phosphate-disodium hydrogen phosphate | 6 | Clarified | 98.6% | Clarified | 95.9% |
| Tris-hydrochloride | 7 | Clarified | 98.4% | Clarified | 96.2% |
| Histidine-acetic acid | 6 | Clarified | 98.5% | Clarified | 96.5% |
| Phosphoric acid-sodium citrate | 6 | Clarified | 98.5% | Clarified | 95.9% |

As can be seen from Tables 6 and 7, the Tm and Tagg values in various buffer systems had little difference. As can be seen from Tables 8 and 9, there was no significant difference among groups in purity under the freeze-thaw pressure condition; under the high-temperature pressure condition, all buffer system groups had no significant difference in appearance and purity.

Subsequently, histidine-acetic acid (pH 6) was selected as the buffer for screening of other excipients.

### Example 7. Stability Comparison of Different Stabilizers

The antibody was exchanged into a histidine-acetic acid (pH 6) buffer system by using a WaterSep hollow fiber (Discover 12), and a stock solution at a high concentration of each stabilizer was added to prepare each stabilizer screening group (as shown in Table 10). The antibody concentration was adjusted to about 30 mg/mL, and the mixture was sterilized and filtered for later use.

The freshly prepared samples were subjected to various pressurization conditions, including: high temperature test (40 °C±2 °C, being left to stand for three weeks) and repeated freeze-thaw (5 cycles, ≤ -70 °C/5 °C±3 °C). The stability was assessed by detecting items such as appearance and purity. Some of the detection results are shown in Tables 10 and 11 below.

**Table 10. Summary of appearance and purity results for 900698 antibody in different stabilizer systems under various pressure conditions**

| Sample | | Freeze-thaw | | High temperature | | |
|---|---|---|---|---|---|---|
| Type | Content | Appearance | SEC purity | Appearance | SEC purity | Main peak of CEX |
| Sucrose | 8 wt.% | Clarified | 98.4% | Clarified | 97.2% | 55.9% |
| Trehalose | 8 wt.% | Clarified | 98.4% | Clarified | 97.4% | 55.1% |
| Sorbitol | 4 wt.% | Clarified | 98.4% | Clarified | 97.4% | 55.5% |
| Mannitol | 4 wt.% | Clarified | 97.1% | Clarified | 97.2% | 56.5% |
| Proline/sucrose | 1.5 wt.%/5 wt.% | Clarified | 98.4% | Clarified | 97.4% | 55.9% |
| Methionine/trehalose | 0.15 wt.%/8 wt.% | Clarified | 98.4% | Clarified | 97.3% | 56.7% |
| Glycine/mannitol | 1 wt.%/4 wt.% | Clarified | 98.5% | Clarified | 97.3% | 55.4% |
| Sodium chloride/sucrose | 0.6 wt.%/5 wt.% | Clarified | 98.4% | Clarified | 96.6% | 56.7% |
| Arginine/sucrose | 1.5 wt.%/5 wt.% | Clarified | 98.4% | Clarified | 97.2% | 56.3% |
| Lysine/trehalose | 0.5 wt.%/7 wt.% | Clarified | 98.3% | Clarified | 96.7% | 52.8% |

**Table 11. Summary of appearance and purity results for 900725 antibody in different stabilizer systems under various pressure conditions**

| Sample | | Freeze-thaw | | High temperature | | |
|---|---|---|---|---|---|---|
| Type | Content | Appearance | SEC purity | Appearance | SEC purity | Main peak |

| | | | | | | of CEX |
|---|---|---|---|---|---|---|
| Sucrose | 8 wt.% | Clarified | 98.4% | Clarified | 97.2% | 57.6% |
| Trehalose | 8 wt.% | Clarified | 98.4% | Clarified | 97.0% | 57.5% |
| Sorbitol | 4 wt.% | Clarified | 98.6% | Clarified | 97.0% | 57.5% |
| Mannitol | 4 wt.% | Clarified | 96.8% | Clarified | 97.0% | 57.7% |
| Proline/sucrose | 1.5 wt.%/5 wt.% | Clarified | 98.4% | Clarified | 97.1% | 57.6% |
| Methionine/trehalose | 0.15 wt.%/8 wt.% | Clarified | 98.4% | Clarified | 97.3% | 58.1% |
| Glycine/mannitol | 1 wt.%/4 wt.% | Clarified | 98.4% | Clarified | 97.0% | 56.4% |
| Sodium chloride/sucrose | 0.6 wt.%/5 wt.% | Clarified | 98.2% | Clarified | 96.6% | 57.7% |
| Arginine/sucrose | 1.5 wt.%/5 wt.% | Clarified | 98.3% | Clarified | 97.2% | 58.1% |
| Lysine/trehalose | 0.5 wt.%/7 wt.% | Clarified | 98.3% | Clarified | 96.6% | 56.9% |

As can be seen from Tables 10 and 11, except that the SEC purity of the mannitol group was slightly lower than that of other groups, there was no significant difference among the other groups under the freeze-thaw pressure; under the high-temperature pressure, the SEC purity of the sodium chloride-sucrose group and the SEC purity of the lysine-trehalose group were slightly lower than that of other groups, and there was no significant difference among the other groups.

Subsequently, sucrose was selected as the stabilizer for performing a surfactant screening experiment.

### Example 8. Surfactant Screening

The anti-CD73 cocktail antibody was exchanged into a buffer system of histidine-acetic acid (pH 6) and 8 wt.% sucrose by using a WaterSep hollow fiber (Discover 12), and a stock solution at a high concentration of each surfactant was added to prepare each surfactant screening group (as shown in Table 12). The antibody concentration was adjusted to about 30 mg/mL, and the mixture was sterilized and filtered for later use.

The freshly prepared samples were placed under the shaking pressurization condition and shaken at room temperature for 3 days (200 rpm). The protective effects of the surfactants Tween-20 (PS-20) and Tween-80 (PS-80) were evaluated by appearance and the number of insoluble particles. Some of the detection results are shown in Table 12 below.

**Table 12. Summary of the number of particles in different surfactants under the shaking pressure condition**

| Formula information | | | Shaking | | |
|---|---|---|---|---|---|
| Basic formula | Surfactant | | Appearance | ≥ 10 µm (particles/mL) | ≥ 25 µm (particles/mL) |
| | Type | Concentration | | | |
| Histidine-acetic acid, containing 8 wt.% sucrose, 30 mg/mL antibody | Absent | / | Opalescent+++ | 230 | 40 |
| | PS-20 | 0.005% | Clarified | 60 | 10 |
| | | 0.01% | Clarified | 20 | 0 |
| | | 0.03% | Opalescent++ | 0 | 0 |
| | | 0.05% | Opalescent++ | 380 | 0 |
| | | 0.1% | Opalescent++ | 110 | 0 |
| | | 0.005% | Clarified | 50 | 0 |
| | | 0.01% | Clarified | 0 | 0 |
| | PS-80 | 0.03% | Opalescent++ | 260 | 40 |
| | | 0.05% | Opalescent++ | 280 | 10 |
| | | 0.1% | Opalescent+ | 80 | 0 |

As can be seen from Table 12, under the shaking pressure, the addition of PS-20 and PS-80 significantly inhibited the increase in the number of insoluble particles caused by shaking, as compared with the group without the addition of the surfactant. For the appearance, "+" refers to the degree of opalescence, wherein "+++" indicates that the opalescence was severe, "++" indicates that the opalescence was relatively severe, and "+" indicates that the opalescence was slight. From the appearance, the addition of PS-20 and PS-80 could both improve the severity of the opalescence in the samples. However, when the concentration of PS-20 and PS-80 exceeded 0.05%, the improvement diminished.

### Example 9. Preparation of Anti-CD73 Cocktail Antibody Formulations in Different Buffer Systems

From Tables 6-12, it can be seen that in the components, except for a few slightly poorer results, there were no significant differences among the other groups.

In conclusion, several groups of buffer systems were selected for pressurization, acceleration, and long-term stability investigation. The buffer systems are as follows:
1) 20 mM histidine-acetic acid, 8 wt.% sucrose, and 0.03 wt.% Tween-80, with a pH of 6±0.5;
2) 20 mM sodium acetate-acetic acid, 8 wt.% trehalose, and 0.03 wt.% Tween-80, with a pH of 6±0.5;
3) 20 mM histidine-acetic acid, 1.5 wt.% proline, 5 wt.% sucrose, and 0.03 wt.% Tween-80, with a pH of 6±0.5; and
4) 20 mM sodium acetate-acetic acid, 0.15 wt.% methionine, 8 wt.% trehalose, and 0.03 wt.% Tween-80, with a pH of 6±0.5.

The anti-CD73 cocktail antibody was first exchanged into each buffer system by using a 30 KD ultrafiltration membrane (PLCTK) from Merck, and the antibody concentration was adjusted to about 30±3 mg/mL. The mixture was sterilized, filtered, and aseptically aliquoted into 1.5 mL EP tubes, one for each system formulation at each time point, 1 mL per tube.

### Example 10. Stability of Anti-CD73 Cocktail Antibody Formulations

In Example 10, the stability of the anti-CD73 cocktail antibody pharmaceutical formulations in these groups of buffer systems prepared in Example 9 was investigated. The stability investigation includes pressurization, acceleration, and long-term stability investigation as follows:

### 10.1 Stability test under pressurization conditions

The pressurization conditions include: high temperature for 3 weeks (40 °C±2 °C), shaking at room temperature for 3 days (60 rmp), repeated freeze-thaw (5 cycles, freeze-thaw at ≤ -70 °C/5 °C±3 °C), etc.

Several groups of anti-CD73 cocktail antibody pharmaceutical formulation samples prepared according to Example 9 were stored under various pressure conditions (as shown in Table 13), collected, and sent for detection after a set time point or number of treatments. The samples were evaluated for stability by appearance, the number of insoluble particles, purity, etc. Some of the detection results are shown in Table 13 below.

**Table 13. Summary of stability data for anti-CD73 cocktail antibody pharmaceutical formulations under various pressurization conditions**

| Sample | Pressurization conditions | Appearance | Insoluble particle | | Purity | | |
|---|---|---|---|---|---|---|---|
| | | | ≥ 10 µm (particles/mL) | ≥ 25 µm (particles/mL) | SEC purity | Main peak of CEX 38 | Main peak of CEX 39 |
| Buffer system 1 | T0 control | Clarified | 140 | 30 | 97.8% | 25.3% | 39.5% |
| | Shaking at room temperature for 3 days | Clarified | 260 | 40 | 97.7% | 26.1% | 40.6% |
| | High temperature for 3 weeks | Clarified | 110 | 65 | 96.4% | 21.0% | 30.5% |
| | Freeze-thaw for 5 cycles | Clarified | / | / | 97.8% | 24.0% | 37.4% |
| Buffer system 2 | T0 control | Clarified | 90 | 10 | 97.3% | 27.1% | 37.1% |
| | Shaking at room temperature for 3 days | Clarified | 60 | 0 | 97.6% | 28.2% | 37.3% |
| | High temperature for 3 weeks | Clarified | 30 | 20 | 95.6% | 20.7% | 26.2% |
| | Freeze-thaw for 5 cycles | Clarified | / | / | 97.5% | 26.9% | 36.7% |
| Buffer system 3 | T0 control | Clarified | 60 | 10 | 97.6% | 25.5% | 40.9% |
| | Shaking at room temperature for 3 days | Clarified | 111 | 0 | 97.5% | 25.6% | 41.5% |
| | High temperature for 3 weeks | Clarified | 25 | 5 | 96.0% | 19.0% | 28.4% |
| | Freeze-thaw for 5 cycles | Clarified | / | / | 97.7% | 25.2% | 40.5% |
| Buffer system 4 | T0 control | Clarified | 50 | 20 | 97.3% | 26.5% | 33.9% |
| | Shaking at room temperature for 3 days | Clarified | 30 | 0 | 97.1% | 28.3% | 35.5% |
| | High temperature for 3 weeks | Clarified | 45 | 10 | 95.8% | 21.3% | 25.4% |
| | Freeze-thaw for 5 cycles | Clarified | / | / | 97.5% | 26.4% | 33.7% |

| | | | | | | | |
|---|---|---|---|---|---|---|---|
| Note: T0 control was the control at the completion of the preparation of the formulation. | | | | | | | |

As can be seen from Table 13, the influence items and degrees on the product quality were different under the pressure conditions. The shaking and high-temperature pressure conditions mainly caused an increase in the number of large particles in the insoluble particles, with a slight decrease in purity, but well above the quality criteria. In summary, the pharmaceutical compositions in these groups of buffer systems have high stability, and can effectively protect the main drug component (anti-CD73 cocktail antibody), thereby effectively resisting the destructive effects of the pressurization conditions.

### 10.2 Accelerated stability test

The anti-CD73 cocktail antibody pharmaceutical formulation samples in these groups of buffer systems prepared according to Example 9 were stored under an acceleration condition (25 °C±2 °C), collected, and sent for detection after a set time point. The samples were evaluated for stability by insoluble particles, purity, etc. Some of the detection results are shown in Table 14 below.

**Table 14. Summary of stability data for anti-CD73 cocktail antibody pharmaceutical formulations under the acceleration condition**

| Sample name | Conditions | Insoluble particle | | Purity | | |
|---|---|---|---|---|---|---|
| | | ≥ 10 µm (particles/mL) | ≥ 25 µm (particles/mL) | SEC purity | Main peak of CEX (38) | Main peak of CEX (39) |
| Buffer system 1 | T0 control | 140 | 30 | 97.8% | 25.3% | 39.5% |
| | 3 weeks | 20 | 10 | 97.6% | 25.3% | 39.0% |
| | Two months | 160 | 10 | 97.3% | 22.8% | 33.9% |
| | Three months | 0 | 0 | 96.8% | 22.1% | 32.2% |
| Buffer system 2 | T0 control | 90 | 10 | 97.3% | 27.1% | 37.1% |
| | 3 weeks | 25 | 15 | 97.2% | 25.5% | 34.4% |
| | Two months | 60 | 0 | 96.7% | 24.3% | 31.5% |
| | Three months | 0 | 0 | 95.8% | 23.4% | 29.8% |
| Buffer system 3 | T0 control | 60 | 10 | 97.6% | 25.5% | 40.9% |
| | 3 weeks | 111 | 0 | 97.4% | 23.3% | 37.2% |
| | Two months | 25 | 5 | 97.1% | 22.2% | 33.7% |
| | Three months | 0 | 0 | 96.2% | 21.1% | 31.3% |
| Buffer system 4 | T0 control | 50 | 20 | 97.3% | 26.5% | 33.9% |
| | 3 weeks | 40 | 15 | 97.2% | 27.5% | 34.6% |
| | Two months | 220 | 0 | 96.9% | 25.0% | 30.8% |
| | Three months | 0 | 0 | 95.9% | 24.1% | 29.2% |

| | | | | | | |
|---|---|---|---|---|---|---|
| Note: T0 control was the control at the completion of the preparation of the formulation. | | | | | | |

As can be seen from Table 14, the acceleration condition caused a certain degree of decline in the quality of the pharmaceutical formulations. In these groups of buffer systems, the stability of 20HA6Arg1.5Suc5ps0.03 was slightly worse, and compared with the T0 sample, the SEC molecular sieve purity was reduced by about 1.4%, the main peak content of CEX (38) was reduced by about 4.4%, and the main peak content of CEX (39) was reduced by about 9.6% in the sample accelerated for 3 months, which, however, still met the quality criteria. In summary, the pharmaceutical compositions in these groups of buffer systems have high stability, and can be stably stored for at least more than 3 months under the acceleration condition at 25 °C.

### 10.3 Long-term stability test

The anti-CD73 cocktail antibody pharmaceutical formulation samples in these groups of buffer systems prepared according to Example 9 were stored under a long-term refrigeration condition (5 °C±3 °C), collected, and sent for detection after a set time point. The samples were evaluated for stability by insoluble particles, purity, etc. Some of the detection results are shown in Table 15 below.

**Table 15. Summary of stability data for anti-CD73 cocktail antibody pharmaceutical formulations under the long-term refrigeration condition**

| Sample name | Conditions | Insoluble particle | | Purity | | |
|---|---|---|---|---|---|---|
| | | ≥ 10 µm (particles/mL) | ≥ 25 µm (particles/mL) | SEC purity | Main peak of CEX (38) | Main peak of CEX (39) |
| Buffer system 1 | T0 control | 140 | 30 | 97.8% | 25.3% | 39.5% |
| | Three months | 0 | 0 | 97.3% | 24.4% | 37.3% |
| Buffer system 2 | T0 control | 90 | 10 | 97.3% | 27.1% | 37.1% |
| | Three months | 10 | 0 | 96.9% | 25.9% | 34.5% |
| Buffer system 3 | T0 control | 60 | 10 | 97.6% | 25.5% | 40.9% |
| | Three months | 10 | 0 | 97.2% | 23.9% | 37.8% |
| Buffer system 4 | T0 control | 50 | 20 | 97.3% | 26.5% | 33.9% |
| | Three months | 40 | 10 | 97.0% | 26.8% | 33.6% |

| | | | | | | |
|---|---|---|---|---|---|---|
| Note: T0 control was the control at the completion of the preparation of the formulation. | | | | | | |

As can be seen from Table 15, the stability of the pharmaceutical formulations in these groups of buffer systems was very good under the long-term refrigeration condition (5 °C±3 °C), and the three-month samples had no significant change in insoluble particles, and had a slight reduction in the purity, which was, however, well above the quality criteria. In summary, the pharmaceutical compositions in these groups of buffer systems have high stability, and can be stably stored under the long-term refrigeration condition.

In conclusion, it can be seen that the anti-CD73 cocktail antibody pharmaceutical formulations in these groups of buffer systems prepared in Example 9 have very good stability, and the anti-CD73 cocktail antibody maintains relatively good stability under various pressure conditions (high temperature, repeated freeze-thaw, and shaking), can be stably stored for at least more than three months under the acceleration condition (25 °C±2 °C), shows high stability under the long-term refrigeration condition at the same time, and can be stably stored.

## Claims

1. A pharmaceutical combination, comprising a first antigen-binding protein that specifically binds to CD73, and a second antigen-binding protein that specifically binds to CD73, wherein the first antigen-binding protein and the second antigen-binding protein have different CD73 antigen-binding epitopes.

2. The pharmaceutical combination according to claim 1, wherein the first antigen-binding protein and the second antigen-binding protein bind to the CD73 protein in a non-competitive manner.

3. The pharmaceutical combination according to any one of claims 1-2, wherein the first antigen-binding protein and the second antigen-binding protein are present in the form of a mixture.

4. The pharmaceutical combination according to any one of claims 1-3, wherein the first antigen-binding protein comprises at least one CDR in an antibody heavy chain variable region VH, the VH comprising the amino acid sequence set forth in SEQ ID NO: 8.

5. The pharmaceutical combination according to any one of claims 1-4, wherein the first antigen-binding protein comprises HCDR3, the HCDR3 comprising the amino acid sequence set forth in SEQ ID NO: 1.

6. The pharmaceutical combination according to any one of claims 1-5, wherein the first antigen-binding protein comprises HCDR2, the HCDR2 comprising the amino acid sequence set forth in SEQ ID NO: 2.

7. The pharmaceutical combination according to any one of claims 1-6, wherein the first antigen-binding protein comprises HCDR1, the HCDR1 comprising the amino acid sequence set forth in SEQ ID NO: 3.

8. The pharmaceutical combination according to any one of claims 1-7, wherein the first antigen-binding protein comprises a VH, the VH comprising the amino acid sequence set forth in SEQ ID NO: 8.

9. The pharmaceutical combination according to any one of claims 1-8, wherein the first antigen-binding protein comprises an antibody heavy chain constant region.

10. The pharmaceutical combination according to claim 9, wherein the antibody heavy chain constant region is derived from an IgG heavy chain constant region.

11. The pharmaceutical combination according to any one of claims 9-10, wherein the antibody heavy chain constant region is derived from a human IgG heavy chain constant region.

12. The pharmaceutical combination according to any one of claims 9-11, wherein the antibody heavy chain constant region comprises the amino acid sequence set forth in SEQ ID NO: 9.

13. The pharmaceutical combination according to any one of claims 1-12, wherein the first antigen-binding protein comprises an antibody heavy chain, the antibody heavy chain comprising the amino acid sequence set forth in SEQ ID NO: 10.

14. The pharmaceutical combination according to any one of claims 1-13, wherein the first antigen-binding protein comprises at least one CDR in an antibody light chain variable region VL, the VL comprising the amino acid sequence set forth in SEQ ID NO: 18.

15. The pharmaceutical combination according to any one of claims 1-14, wherein the first antigen-binding protein comprises LCDR3, the LCDR3 comprising the amino acid sequence set forth in SEQ ID NO: 11.

16. The pharmaceutical combination according to any one of claims 1-15, wherein the first antigen-binding protein comprises LCDR2, the LCDR2 comprising the amino acid sequence set forth in SEQ ID NO: 12 (WAS).

17. The pharmaceutical combination according to any one of claims 1-16, wherein the first antigen-binding protein comprises LCDR1, the LCDR1 comprising the amino acid sequence set forth in SEQ ID NO: 13.

18. The pharmaceutical combination according to any one of claims 1-17, wherein the first antigen-binding protein comprises a VL, the VL comprising the amino acid sequence set forth in SEQ ID NO: 18.

19. The pharmaceutical combination according to any one of claims 1-18, wherein the first antigen-binding protein comprises an antibody light chain constant region.

20. The pharmaceutical combination according to claim 19, wherein the antibody light chain constant region is derived from a human Igκ constant region.

21. The pharmaceutical combination according to any one of claims 19-20, wherein the antibody light chain constant region comprises the amino acid sequence set forth in SEQ ID NO: 19.

22. The pharmaceutical combination according to any one of claims 1-21, wherein the first antigen-binding protein comprises an antibody light chain, the antibody light chain comprising the amino acid sequence set forth in SEQ ID NO: 20.

23. The pharmaceutical combination according to any one of claims 1-22, wherein the second antigen-binding protein comprises at least one CDR in an antibody heavy chain variable region VH, the VH comprising the amino acid sequence set forth in SEQ ID NO: 27.

24. The pharmaceutical combination according to any one of claims 1-23, wherein the second antigen-binding protein comprises HCDR3, the HCDR3 comprising the amino acid sequence set forth in SEQ ID NO: 21.

25. The pharmaceutical combination according to any one of claims 1-24, wherein the second antigen-binding protein comprises HCDR2, the HCDR2 comprising the amino acid sequence set forth in SEQ ID NO: 22.

26. The pharmaceutical combination according to any one of claims 1-25, wherein the second antigen-binding protein comprises HCDR1, the HCDR1 comprising the amino acid sequence set forth in SEQ ID NO: 23.

27. The pharmaceutical combination according to any one of claims 1-26, wherein the second antigen-binding protein comprises a VH, the VH comprising the amino acid sequence set forth in SEQ ID NO: 27.

28. The pharmaceutical combination according to any one of claims 1-27, wherein the second antigen-binding protein comprises an antibody heavy chain constant region.

29. The pharmaceutical combination according to claim 28, wherein the antibody heavy chain constant region is derived from an IgG heavy chain constant region.

30. The pharmaceutical combination according to any one of claims 28-29, wherein the antibody heavy chain constant region is derived from a human IgG heavy chain constant region.

31. The pharmaceutical combination according to any one of claims 28-30, wherein the antibody heavy chain constant region comprises the amino acid sequence set forth in SEQ ID NO: 9.

32. The pharmaceutical combination according to any one of claims 1-31, wherein the second antigen-binding protein comprises an antibody heavy chain, the antibody heavy chain comprising the amino acid sequence set forth in SEQ ID NO: 28.

33. The pharmaceutical combination according to any one of claims 1-32, wherein the second antigen-binding protein comprises at least one CDR in an antibody light chain variable region VL, the VL comprising the amino acid sequence set forth in SEQ ID NO: 35.

34. The pharmaceutical combination according to any one of claims 1-33, wherein the second antigen-binding protein comprises LCDR3, the LCDR3 comprising the amino acid sequence set forth in SEQ ID NO: 29.

35. The pharmaceutical combination according to any one of claims 1-34, wherein the second antigen-binding protein comprises LCDR2, the LCDR2 comprising the amino acid sequence set forth in SEQ ID NO: 30 (LAS).

36. The pharmaceutical combination according to any one of claims 1-35, wherein the second antigen-binding protein comprises LCDR1, the LCDR1 comprising the amino acid sequence set forth in SEQ ID NO: 31.

37. The pharmaceutical combination according to any one of claims 1-36, wherein the second antigen-binding protein comprises a VL, the VL comprising the amino acid sequence set forth in SEQ ID NO: 35.

38. The pharmaceutical combination according to any one of claims 1-37, wherein the second antigen-binding protein comprises an antibody light chain constant region.

39. The pharmaceutical combination according to claim 38, wherein the antibody light chain constant region is derived from a human Igκ constant region.

40. The pharmaceutical combination according to any one of claims 38-39, wherein the antibody light chain constant region comprises the amino acid sequence set forth in SEQ ID NO: 19.

41. The pharmaceutical combination according to any one of claims 1-40, wherein the second antigen-binding protein comprises an antibody light chain, the antibody light chain comprising the amino acid sequence set forth in SEQ ID NO: 36.

42. The pharmaceutical combination according to any one of claims 1-41, wherein the first antigen-binding protein and the second antigen-binding protein are in a mass ratio of about 1:5 to 5:1.

43. The pharmaceutical combination according to any one of claims 1-42, wherein the first antigen-binding protein and the second antigen-binding protein are in a mass ratio of about 1:5.

44. The pharmaceutical combination according to any one of claims 1-42, wherein the first antigen-binding protein and the second antigen-binding protein are in a mass ratio of about 1:2.5.

45. The pharmaceutical combination according to any one of claims 1-42, wherein the first antigen-binding protein and the second antigen-binding protein are in a mass ratio of about 1:1.

46. The pharmaceutical combination according to any one of claims 1-42, wherein the first antigen-binding protein and the second antigen-binding protein are in a mass ratio of about 2.5:1.

47. The pharmaceutical combination according to any one of claims 1-42, wherein the first antigen-binding protein and the second antigen-binding protein are in a mass ratio of about 5:1.

48. A pharmaceutical composition, comprising the pharmaceutical combination according to any one of claims 1-47, and optionally one or more pharmaceutically acceptable carriers.

49. The pharmaceutical composition according to claim 48, wherein the pharmaceutical combination is at a concentration of about 3-300 mg/mL.

50. The pharmaceutical composition according to any one of claims 48-49, wherein the pharmaceutical combination is at a concentration of about 3-150 mg/mL.

51. The pharmaceutical composition according to any one of claims 48-50, wherein the pharmaceutical combination is at a concentration of about 5-100 mg/mL.

52. The pharmaceutical composition according to any one of claims 48-51, wherein the pharmaceutical combination is at a concentration of about 10-100 mg/mL.

53. The pharmaceutical composition according to any one of claims 48-52, wherein the pharmaceutical combination is at a concentration of about 10-50 mg/mL.

54. The pharmaceutical composition according to any one of claims 48-53, wherein the pharmaceutical combination is at a concentration of 30±5 mg/mL.

55. The pharmaceutical composition according to any one of claims 48-54, wherein the pharmaceutical combination is at a concentration of 30±3 mg/mL.

56. The pharmaceutical composition according to any one of claims 48-55, wherein the pharmaceutically acceptable carrier comprises a buffer.

57. The pharmaceutical composition according to claim 56, wherein the buffer comprises an acidic buffer.

58. The pharmaceutical composition according to any one of claims 56-57, wherein the buffer comprises a neutral buffer.

59. The pharmaceutical composition according to any one of claims 56-58, wherein the buffer is selected from the group consisting of: an acetate-acetic acid buffer, a histidine-histidine hydrochloride buffer, a citrate-citric acid buffer, a phosphate buffer, a histidine-acetic acid buffer, a tris(hydroxymethyl)aminomethane (Tris)-hydrochloride buffer, phosphoric acid-citrate buffer , and a combination thereof.

60. The pharmaceutical composition according to any one of claims 56-59, wherein the buffer is selected from the group consisting of: acetate-acetic acid, citrate-citric acid, histidine-histidine hydrochloride, phosphate, Tris-hydrochloride, histidine-acetic acid, phosphoric acid-citrate, and a combination thereof.

61. The pharmaceutical composition according to any one of claims 56-60, wherein the buffer is selected from the group consisting of: sodium acetate-acetic acid, sodium citrate-citric acid, histidine-histidine hydrochloride, sodium dihydrogen phosphate-disodium hydrogen phosphate, Tris-hydrochloride, histidine-acetic acid, and phosphoric acid-sodium citrate.

62. The pharmaceutical composition according to any one of claims 56-61, wherein the buffer is at a concentration of about 5-100 mM.

63. The pharmaceutical composition according to any one of claims 56-62, wherein the buffer is at a concentration of about 5-60 mM.

64. The pharmaceutical composition according to any one of claims 56-63, wherein the buffer is at a concentration of about 5-50 mM.

65. The pharmaceutical composition according to any one of claims 56-64, wherein the buffer is at a concentration of about 10-40 mM.

66. The pharmaceutical composition according to any one of claims 56-65, wherein the buffer is at a concentration of 20±5 mM.

67. The pharmaceutical composition according to any one of claims 56-66, wherein the buffer is at a concentration of 20±2 mM.

68. The pharmaceutical composition according to any one of claims 48-67, wherein the pharmaceutical composition has a pH value of about 5-7.

69. The pharmaceutical composition according to any one of claims 48-68, wherein the pharmaceutical composition has a pH value of about 6±0.5.

70. The pharmaceutical composition according to any one of claims 48-69, wherein the pharmaceutical composition has a pH value of about 6±0.3.

71. The pharmaceutical composition according to any one of claims 48-70, wherein the pharmaceutical composition has a pH value of about 6+0.1.

72. The pharmaceutical composition according to any one of claims 48-68, wherein the pharmaceutical composition has a pH value of about 6.5±0.3.

73. The pharmaceutical composition according to any one of claims 48-68, wherein the pharmaceutical composition has a pH value of about 7±0.3.

74. The pharmaceutical composition according to any one of claims 48-73, wherein the pharmaceutically acceptable carrier comprises a stabilizer.

75. The pharmaceutical composition according to claim 74, wherein the stabilizer is selected from the group consisting of: sodium chloride, an amino acid, a sugar alcohol, and a combination thereof.

76. The pharmaceutical composition according to claim 75, wherein the amino acid is selected from the group consisting of: proline, arginine, glycine, histidine, lysine, methionine, and a combination thereof.

77. The pharmaceutical composition according to any one of claims 75-76, wherein the sugar alcohol is selected from the group consisting of: sucrose, mannitol, trehalose, sorbitol, and a combination thereof.

78. The pharmaceutical composition according to any one of claims 74-77, wherein the stabilizer is selected from the group consisting of: sucrose, trehalose, sorbitol, mannitol, proline/sucrose, methionine/trehalose, glycine/mannitol, sodium chloride/sucrose, arginine/sucrose, and lysine/trehalose.

79. The pharmaceutical composition according to any one of claims 74-78, wherein the content of the stabilizer is about 0.15 wt.%-20 wt.%.

80. The pharmaceutical composition according to any one of claims 74-79, wherein the content of the stabilizer is about 0.15 wt.%-10 wt.%.

81. The pharmaceutical composition according to any one of claims 74-80, wherein the content of the stabilizer is about 0.15 wt.%-8 wt.%.

82. The pharmaceutical composition according to any one of claims 74-81, wherein the content of the stabilizer is about 4±1 wt.% or 8±1 wt.%.

83. The pharmaceutical composition according to any one of claims 74-82, wherein the content of the stabilizer is about 4±0.5 wt.% or 8±0.5 wt.%.

84. The pharmaceutical composition according to any one of claims 74-81, wherein the content of the stabilizer is about 4 wt.%, 5 wt.%, 7 wt.%, or 8 wt.%.

85. The pharmaceutical composition according to any one of claims 74-84, wherein the content of the stabilizer is about 8 wt.%.

86. The pharmaceutical composition according to any one of claims 48-85, wherein the pharmaceutically acceptable carrier comprises a surfactant.

87. The pharmaceutical composition according to claim 86, wherein the surfactant is selected from the group consisting of: a polyoxyethylene sorbitan fatty acid ester, polyoxyethylene hydrogenated castor oil,, a glycerol fatty acid ester, polysorbate, poloxamer, and a combination thereof.

88. The pharmaceutical composition according to claim 87, wherein the polysorbate is selected from the group consisting of: polysorbate 20 (PS-20, Tween-20), polysorbate 40 (PS-40), polysorbate 60 (PS-60), and polysorbate 80 (PS-80).

89. The pharmaceutical composition according to any one of claims 86-88, wherein the surfactant is selected from the group consisting of: PS-20 (Tween-20) and PS-80 (Tween-80).

90. The pharmaceutical composition according to any one of claims 86-89, wherein the surfactant is at a concentration of about 0.001%-0.5%.

91. The pharmaceutical composition according to any one of claims 86-90, wherein the surfactant is at a concentration of about 0.005%-0.1%.

92. The pharmaceutical composition according to any one of claims 86-91, wherein the surfactant is at a concentration of about 0.005%, 0.01%, 0.03%, 0.05%, or 0.1%.

93. The pharmaceutical composition according to any one of claims 86-92, wherein the surfactant is at a concentration of about 0.01%.

94. The pharmaceutical composition according to any one of claims 86-92, wherein the surfactant is at a concentration of about 0.03%.

95. The pharmaceutical composition according to any one of claims 48-94, wherein the pharmaceutically acceptable carrier is selected from the group consisting of:
(1) 20 mM histidine-acetic acid, 8 wt.% sucrose, and 0.03 wt.% Tween-80;
(2) 20 mM sodium acetate-acetic acid, 8 wt.% trehalose, and 0.03 wt.% Tween-80;
(3) 20 mM histidine-acetic acid, 1.5 wt.% proline, 5 wt.% sucrose, and 0.03 wt.% Tween-80;
(4) 20 mM sodium acetate-acetic acid, 0.15 wt.% methionine, 8 wt.% trehalose, and 0.03 wt.% Tween-80;
(5) 20 mM histidine-acetic acid, 8 wt.% sucrose, and 0.01 wt.% Tween-80;
(6) 20 mM sodium acetate-acetic acid, 8 wt.% trehalose, and 0.01 wt.% Tween-80;
(7) 20 mM histidine-acetic acid, 1.5 wt.% proline, 5 wt.% sucrose, and 0.01 wt.% Tween-80; and
(8) 20 mM sodium acetate-acetic acid, 0.15 wt.% methionine, 8 wt.% trehalose, and 0.01 wt.% Tween-80.

96. Use of the pharmaceutical combination according to any one of claims 1-47 and/or the pharmaceutical composition according to any one of claims 48-95 in the preparation of a medicament for preventing and/or treating diseases and/or disorders.

97. The use according to claim 96, wherein the diseases and/or disorders comprises a CD73-mediated disease and/or condition.

98. The use according to any one of claims 96-97, wherein the diseases and/or disorders comprises a tumor.

99. The use according to claim 98, wherein the tumor comprises a solid tumor and/or a hematological tumor.
100.The use according to any one of claims 96-99, wherein the diseases and/or disordersis selected from the group consisting of: pancreatic cancer, breast cancer, and idiopathic pulmonary fibrosis (IPF).
101.The use according to claim 100, wherein the breast cancer comprises triple-negative breast cancer.
102.Use of the pharmaceutical combination according to any one of claims 1-47 and/or the pharmaceutical composition according to any one of claims 48-95 in the preparation of a detection kit for detecting the presence and/or content of CD73 protein.
